# EUROPEAN PATENT SPECIFICATION

(11) **EP 3 066 213 B1**
(45) Date of publication and mention of the grant of the patent: **06.11.2019**
(21) Application number: 14802610.7
(22) Date of filing: 10.11.2014
(51) Int. Cl.: C12Q 1/6869

(54) **GENETIC ANALYSIS METHOD**
GENETISCHES ANALYSEVERFAHREN
PROCÉDÉ D'ANALYSE GÉNÉTIQUE

(30) Priority: 08.11.2013 GB 201319779
(43) Date of publication of application: 14.09.2016
(73) Proprietor: Agilent Technologies Belgium NV, 1831 Machelen (BE)
(72) Inventor: DEVOGELAERE, Benoit, 1800 Vilvoorde (BE); VERRELST, Herman, 3000 Leuven (BE)
(74) Representative: Gevers Patents
(86) International application number: PCT/EP2014/074155
(87) International publication number: WO 2015/067796

(56) References cited:
- WO-A1-2011/095501
- US-A1- 2012 252 682
- US-A1- 2012 316 073
- BRANT K. PETERSON ET AL: "Double Digest RADseq: An Inexpensive Method for De Novo SNP Discovery and Genotyping in Model and Non-Model Species", PLOS ONE, vol. 7, no. 5, 31 May 2012 (2012-05-31), page e37135, XP055110881, DOI: 10.1371/journal.pone.0037135
- YU ZHANG: "De novo inference of stratification and local admixture in sequencing studies", BMC BIOINFORMATICS, BIOMED CENTRAL, LONDON, GB, vol. 14, no. Suppl 5, 10 April 2013 (2013-04-10), page S17, XP021146341, ISSN: 1471-2105, DOI: 10.1186/1471-2105-14-S5-S17
- JOSEPH B HIATT ET AL: "Parallel, tag-directed assembly of locally derived short sequence reads", NATURE METHODS, vol. 7, no. 2, 1 February 2010 (2010-02-01), pages 119-122, XP055005905, ISSN: 1548-7091, DOI: 10.1038/nmeth.1416
- HUANG X ET AL: "CAP3: A DNA Sequence Assembly Program", GENOME RESEARCH, COLD SPRING HARBOR LABORATORY PRESS, US, vol. 9, 1 January 1999 (1999-01-01), pages 868-877, XP002267210, ISSN: 1088-9051, DOI: 10.1101/GR.9.9.868
- J. R. MILLER ET AL: "Aggressive assembly of pyrosequencing reads with mates", BIOINFORMATICS, vol. 24, no. 24, 24 October 2008 (2008-10-24), pages 2818-2824, XP055217082, ISSN: 1367-4803, DOI: 10.1093/bioinformatics/btn548

## Description

### Field of the invention

The invention relates generally to the field of DNA analysis. More in particular, it applies to the field of data analysis for DNA typing. Processes and systems are described that allow for the quick and reliable interpretation of nucleic acid information.

### Introduction

Next generation sequencing (NGS) has enabled the generation of large-scale genome sequence data. Theoretically, it is possible to detect single nucleotide polymorphisms (SNPs), molecular or copy number variations (CNV) from NGS data. However, whole genome data processing and variant calling from NGS is confronted with a statistical inference problem due to a number of shortcomings in the conventional art.

A number of problems arise from the fact that most of the NGS platforms generate massive amounts of data in the form of short read lengths. The big amount of short read lengths make assembly of the genome difficult and time consuming. Due to the fact that massive amounts of data are created, NGS also encounters data storage and data transfer challenges. Because of the shortness of read lengths, NGS is also confronted with ambiguities in alignment that arise in the areas of repeat DNA.

Further problems arise from the NGS data type input used for further processing. Most statistical methods summarize the NGS data into discrete base calls, discrete polymorphism calls and discrete parental information calls and use this as input information for their further analysis. The application of discrete calls as an input may filter out information applicable in a later stage, such as during downstream analysis requiring data artefact corrections.

In particular settings, the availability of insufficient amounts of sample material may require additional sample handling such as Whole Genome Amplification (WGA) and Partial Genome Amplification (PGA) using multiple displacement amplification (MDA) or PCR-based methods, which will result in NGS data with incomplete loci or incorrect coverage (e.g. allele drop out or preferential amplification of certain genome regions over others).

From the above, it seems there is a continuing need for improved structured ways of sequence data management, data accessibility and reliable computational analyses of sequence data.

EP1951897 (Handyside) discloses a method of karyotyping a target cell to detect chromosomal imbalance(s) therein. The method thereto focuses on the interrogation of closely adjacent bi-allelic SNPs across the chromosome of the target cell and compares the result with the SNP haplotype of paternal and maternal chromosomes to assemble a notional haplotype of the target cell chromosomes of paternal origin and of maternal origin. In a subsequent step, the notional SNP haplotype of target cell chromosomes of paternal origin and of maternal origin are assessed to detect aneuploidy of the chromosome in the target cell or to detect the inheritance of a target allele potentially linked to an inheritable disorder. This method uses informative or semi- informative SNP only as input metric for the analysis.

WO2013/052557 (Natera et al.) describes a method for determining the ploidy status of an embryo at a chromosome from a sample of DNA from an embryo. DNA from one or more cells biopsied from the embryo is amplified at a plurality of loci by targeted amplification, sequenced and the number of sequence reads in the sequence data associated with each of a plurality of loci on the chromosome of interest is counted. The observed number of reads at a particular locus is then compared to the expected number of reads at that particular locus based on reference data to make a conclusion on the ploidy state of the embryo. This method thus compares sequence read count at individual loci obtained for the target sample with sequence read count obtained for the same locus in reference samples. This method does not allow for the diagnosis of risk alleles associated with inheritable disorders.

Two references (Elshire et al. 2011; De Donato et al., 2013) describe genotyping-by-sequencing with use of restriction enzymes to partition the target DNA. Both methods use read numbers and SNP calls as input metric. Elshire et al. describe a genotyping-by-sequencing method that uses methylation-sensitive restriction enzyme digestion to fragment the target DNA, followed by sequencing, and the identification of sequence tags that can be used as markers in high diversity, large genome plants. De Donato et al. describe a genotyping-by-sequencing method that uses restriction enzyme digestion to fragment the target DNA, followed by sequencing, and the identification of SNP markers that can serve as acceptable markers for genomic selection and genome-wide association studies in cattle. Both methods aim to identify markers, and neither of the methods allows to make an analysis in terms of (sub)chromosomal CNV screening, the diagnosis of the presence of risk alleles linked to inheritable disorders, or the diagnosis of the presence of a balanced translocation or inversion.

Peterson et al. (2012) provides a method for generating a reduced representation library for SNP discovery and genotyping in model and non-model species. The generation of the reduced representation library involves digesting genomic DNA with two restriction enzymes, barcoded adaptor ligation, a tight size selection of the ligated fragments followed by sequencing at an average of 10x coverage. However, the method requires relatively large amounts of genomic DNA (at least 100 ng). Furthermore, subsequent analysis of sequencing data requires ploidy-aware filtering. Only putative ortholog sets for which greater than 90% of reads are one of the two most frequent unique sequences are retained for a diploid individual. As such, the method does not allow for genomic DNA analysis in a ploidy-unaware situation, such as for determining aneuploidy. Furthermore, because the method only retains reads containing the two most frequent alleles, it discards valuable information, such as sequencing information for triallelic polymorphisms and sequences with allele drop-in errors. As the method is designed for de novo SNP discovery, it does not rely on mapping observed reads to a reference genome. The method hence is also incompatible with clustering non-overlapping nearby segments derived from the reduced representation library, because the relative and absolute position of the segments in the reference genome is unknown. In fact, the method does not perform any type of similarity-based clustering to remove noise in the genotyping data.

Recently, Zheng et al. (Zheng et al., 2013) described the detection of copy number variation (CNV) using a targeted sequencing technique that involves restriction digestion with a single restriction enzyme, ligation of a first adaptor, sonication to perform random physical shearing of the DNA, size selection, and ligation of a second adaptor to the random shearing-induced breakpoint. The shearing occurs at random locations throughout the genome, and can therefore not be in silico predicted. The DNA is extracted from tumor material and a large amount (2 ug) is used for the enzyme digestion step. Reads are mapped to a small subset of the whole genome, which is composed of flanking regions adjacent to the restriction site.

The method requires the grouping of a fixed number of consecutive restriction sites (no less than 10) to allow for measurement of the dispersion of the read depth profiles. The number of grouped consecutive restriction sites is fixed during the analysis. The method requires the identification of heterozygous sites via a comparison with an adjacent non-tumor sample, in which heterozygous sites
(1) need to be included in the SNP database dbSNP130
(2) the number of sequence reads of that SNP should be no lower than 20
(3) the minor allele frequency of the SNP in the adjacent non-tumour sample should be not lower than 0.3
(4) the interval between 2 SNPs should be at least 10 bp The method requires a large amount of target DNA (2 ug) extracted from the tumour sample and from an adjacent, healthy tissue sample, and hence is not applicable to non-tumour samples, such as in preimplantation genetic testing, or embryo screening. The method is specific for the identification of genomic CNVs and does not allow for the diagnosis of the presence of risk alleles linked to inheritable disorders, or the diagnosis of the presence of balanced translocations and inversions.

US2012/0316073 relates to strategies for high throughput identification and detection of polymorphisms.

Thus, a need remains for improved methods with increased computational and storage efficiency for target DNA genome analysis. In particular for samples wherein low amounts of genomic DNA are available (e.g. only 100 ng or less), such as samples containing only a few cells. Furthermore, for example in the field of preimplantation testing, improved methods for whole genome aneuploidy detection and familial inheritance determination are required.

### Brief description of the invention

It is an objective of the present invention to remedy all or part of the disadvantages mentioned above. The present invention fulfils these objectives by providing methods allowing for the easy and quick interpretation of a genome sequence. In particular, the methods of the present invention allow for a genome-wide analysis with increased computational and storage efficiency and are particularly suitable for samples with low amounts of genomic DNA.

Present invention is defined by the appended claims.

The present disclosure refers to a method of target DNA genome analysis, which method comprises the steps of:
- obtaining raw metrics for non-overlapping segments using a sequencing process applied on a reduced representation library of said target DNA genome,
   wherein said reduced representation library has been enriched for target DNA genome fragments having two boundaries defined by predetermined DNA sequences;
- clustering non-overlapping, nearby segments with similar raw metrics to provide master segments;
- providing metrics describing the master segments in which said metrics include inferred boundaries of one or more master segments, number of observed reads in one or more master segments, observed 4-base frequencies in said one or more master segments, or ancestral probability for one or more of said master segments.

The present disclosure further refers to a method of target DNA genome analysis, which method comprises the steps of:
- obtaining non-overlapping segments of target DNA stretches with segment boundaries defined by the presence of particular restriction enzyme recognition sites, whereby the assembly of said non-overlapping segments compose a reduced representation library of said target DNA genome;
- obtaining for said segments, raw metrics from a sequencing process applied on said reduced representation library;
- clustering non-overlapping, nearby segments with similar raw metrics to provide master segments;
- providing metrics describing the master segments in which said metrics include inferred boundaries of one or more master segments; number of observed reads in one or more master segments, observed base frequencies in said one or more master segments, or ancestral probability for one or more of said master segments.

In one embodiment the raw metrics include anyone of base frequency, read count, ancestral information, or any combinations thereof. In another embodiment the raw metrics as used in the methods of the present invention include anyone of base frequency, read count or the combination thereof. In an even further embodiment the raw metrics as used in the methods of the present invention comprise ancestral information. In one embodiment the raw metrics as used in the methods of the present invention include base frequency, read count, and ancestral information.

In a particular embodiment, the clustering step is based at least on base frequency and read count. In a further embodiment, the clustering step further includes ancestral information. In the methods of the present disclosure the clustering into master segments preferably uses an in silico simulated genome. In one embodiment the clustering into master segments uses pedigree information; in particular ancestral probability-based and derived from pedigree information.

The present methods uses sequencing results from a well defined reduced representation library (RRL) of a genome. Those sequencing results give sufficient leverage to make predictions about typing or ancestral origin in terms of probabilities.

In one embodiment, the methods of the present disclosure may also comprise the step of making a RRL of the target DNA genome and sequencing the RRL of the target genome.

In one embodiment, the methods of the present disclosure may comprise the further step of making a statement on the analysis based on the master segments or master segment associated metrics. In one embodiment the methods of the invention may also comprise the step of making a final discrete DNA call based on the clustering of segments. Such step of making a final discrete DNA call may for example comprise probability-based identification of one or more of; chromosomal recombination sites, (sub)chromosomal copy number variations, deletions, unbalanced or balanced translocations, inversions, amplifications, the presence of risk alleles for inherited disorders, errors in meiosis I or meiosis II, balanced structural chromosome abnormalities; epigenomic profiles of cells, mosaicisms, human leucocyte antigen (HLA) matches, noise typing, copy number, or ancestral origin; in particular involving a probability-based identification of one or more of; chromosomal recombination sites, (sub)chromosomal copy number variations, deletions, unbalanced or balanced translocations, inversions, amplifications, the presence of risk alleles for inherited disorders, errors in meiosis I or meiosis II, balanced structural chromosome abnormalities; epigenomic profiles of cells, mosaicisms, human leucocyte antigen (HLA) matches, or noise typing. In one embodiment the final discrete DNA call involves determining copy number and ancestral origin of the master segments.

In one embodiment, the analysis involves probability-based identification of chromosomal recombination sites; copy number variations such as (sub) chromosomal CNVs, deletions, unbalanced translocations, amplifications, the presence of risk alleles for inherited disorders, non-disjunction errors in meiosis I or meiosis II, balanced structural chromosome abnormalities (such as balanced translocations and inversions), epigenomic profiling of cells, mosaicisms, human leucocyte antigen (HLA) matching, noise typing, or more.

A number of technical advantages are associated to the present methods. By applying RRL, less DNA per sample needs to be sequenced, the NGS run time is reduced and more samples can be pooled in a single run thereby reducing the associated cost.

The present methods rely on the presence of predetermined sequences in the target DNA genome to produce a reduced representation library of said DNA genome. Preferably, the predetermined sequence comprises about 4-8 predetermined bases. In one embodiment, the two boundaries of the target DNA genome fragments are defined by (in particular have) different predetermined sequences. In a particular embodiment, the predetermined sequence is a restriction enzyme recognition site. Said embodiment relies on the presence of restriction enzyme recognition sites to produce a RRL of the target genome. Non-overlapping segments of target DNA stretches with segment boundaries defined by the presence of particular predetermined sequences, e.g. restriction enzyme recognition sites, are assembled to compose a RRL of the target DNA. As will be explained in the detailed description, a number of advantages are associated with the use of predetermined sequences, e.g. restriction enzyme recognition sites, such as the use of a sparse reference genome for read alignment, improved read alignment and directional amplification. This results in a reduced time requirement for data analysis.

In contrast to existing typing methods, the present methods make predictions about typing or ancestral origin based on metrics derived from clustering of segments. The clustering of the segments is based on the use of raw metrics obtained from the sequencing process. More in particular, the present methods use pattern recognition across non-overlapping, nearby segments with similar raw metrics to provide master segments defined by metrics. These metrics can be used in the enhanced interpretation of a target genome as well as in downstream chromosomal analyses such as the identification of the presence of risk alleles for inheritable disorders, balanced and unbalanced translocations or inversions, deletions, amplifications, or (sub)chromosomal copy number variations, or assessments of epigenetic changes of the genome, or the identification of breakpoints or recombination sites, etc....

The combination of the above-mentioned characteristics makes the outcome of the analysis more robust, more efficient and more reliable. The methods are particularly advantageous in applications with limited target DNA availability.

In particular, the present disclosure relates to a method for genome-wide target DNA genome analysis, comprising obtaining a genome-wide reduced representation library as described herein, performing clustering of genome-wide segments as described herein and optionally making genome-wide DNA calls. In a particular embodiment the target DNA used in the target DNA genome analysis methods of the present invention is derived from a small number of cells, e.g from 1 to 1000 cells; in particular from 1 to 10 cells. Thus in a further embodiment the methods of the present invention are used for target DNA genome analysis of target DNA derived from a small number of cells, such as for example target DNA derived from one or two blastomeres, cells from a trophectoderm biopsy, one or two polar bodies, foetal cells or cell-free foetal DNA found in the maternal peripheral blood circulation, or circulating tumour cells or cell-free tumour DNA.

The present invention overcomes shortcomings of the conventional art and may achieve other advantages not contemplated by the conventional methods and systems.

### Brief description of the drawings

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.
**Fig. 1****: Overview of a preferred embodiment of the target DNA genome analysis according to the invention.**
**Fig. 2****: Overview of a preferred embodiment of a method of the invention, from sample preparation to sequencing.**
**Fig. 3****: Overview of preferred embodiments concerning sequencing data processing.** Fig. 3A: Demultiplexing and read mapping of NGS reads containing two different sample-specific barcodes. Fig. 3B: Clustering of segments (diploidy). Fig. 3C: Clustering of segments (triploidy).

### Detailed description

The disclosure can be implemented in numerous ways, including as a process or method; an apparatus; a system; a composition of matter; a computer program product embodied on a computer readable storage medium and/or a processor, such as a processor configured to execute instructions stored on and/or provided by a memory coupled to the processor. In this specification, these implementations, may be referred to as methods.

As used herein, the term "or" is an inclusive "or" operator and is equivalent to the term "and/or" unless the context clearly dictates otherwise. The meaning of "a", "an", and "the" include plural references.

It is an aspect of the present disclosure to provide methods of improved target DNA genome analysis. The methods may be part of a complete service and product, including sequencing parts of a subject's genome; sequence data conversion; data processing; storage of the data; and reporting. Data processing may include steps of de-multiplexing, mapping, counting of reads, variant calling, noise reduction and phasing (when applicable).

The term "subject" or "target" refers to a biological organism such as an individual, a human or other animal (e.g., a pig, a cow, a mouse, etc.) and the like, or a plant, bacterium, archaeon, or virus. In a particular embodiment, the subject or target refers to a mammal, such as a human, a horse, a pig, a cow, etcetera. In some embodiments, any entity having a genotype is a subject, including an embryo (or part thereof), foetus, preimplantation embryo, sperm, egg,... In a preferred embodiment, the target DNA genome is derived from a human subject, such as an embryo, foetus, sperm, egg, or human person.

The methods of target DNA genome analysis use raw metrics obtained by a process of sequencing. DNA sequencing technologies associated with the present invention comprise second, third or fourth generation sequencing techologies including, but not limited to, pyrosequencing (e.g. Roche 454), fluorescence-based sequencing (e.g. Illumina HiSeq, Illumina MiSeq, Pacific Biosciences RS, Pacific Biosciences RSII), proton-based sequencing (Ion Torrent PGM, Ion Torrent Proton), nanopore-based sequencing (Oxford Nanpore Technologies MinION, Oxford Nanopore Technologies GridION), nanowire-based sequencing (QuantuMDx Q-SEQ, QuantuMDx Q-POC).

The sequencing process is applied on a reduced representation library that partitions DNA into sub-regions for sequencing. Reduced representation libraries (RRL) have the advantage of being able to reduce the complexity of a genome by orders of magnitude, with the extent of the reduction being well controllable. With this approach only a fraction of the genome of the sample needs to be sequenced, the run time is reduced and less data storage and transfer capacity is needed. The RRL used in the methods of the present invention is based on the presence of predetermined sequences, such as restriction enzyme recognition sites (RERS). The use of predetermined sequences, such as RERS, provides some benefits compared to other methods. The use of predetermined sequences, such as RERS, enables the production of well-defined genome fragments that will define the molecular entry points for mapping of the sequencing reads. In this way, mapping is facilitated and less storage and analysis capacity is needed as compared to whole genome sequencing. In addition, the use of predetermined sequences, such as RERS, enables directional amplification, thereby increasing the fraction of different-ended fragments and decreasing the fraction of same-ended fragments. Same-ended fragments are generally not desired, as exemplified by e.g. the Illumina sequencing approach, where same-ended fragments can bind to the flowcell, but cannot produce DNA sequence reads. Therefore, in a particular embodiment, the reduced representation library has been enriched for target DNA genome fragments with boundaries defined by two different predetermined DNA sequences.

The present inventors have unexpectedly found that the use of predetermined sequences yields an efficient NGS library. Indeed, enrichment based on 1 or more predetermined sequences will typically yield fragments of which at least a proportion contains the same predetermined sequence at both ends (i.e. so-called same-ended fragments). Hence, it can be expected that attaching adaptors to these same-ended fragments would yield fragments that contain identical adaptors at both ends. Such fragments with identical adaptors at both ends can typically bind to e.g. the flowcell of an Illumina NGS device (or e.g. the bead that is used during emulsion PCR which is used for IonTorrent), but can not be efficiently amplified on certain NGS platforms (e.g. during cluster generation on an Illumina NGS device or) and hence will reduce the amount of usable sequence data that can be generated during the NGS run. In order to overcome this issue, the present invention may at least partially rely on the fact that fragments that contain identical adaptors will not be efficiently amplified during a subsequent PCR step (which is performed after the enrichment for genome fragments with boundaries defined by predetermined sequence and adaptor ligation, but before the pooling of samples and subsequent NGS analysis) because the identical adaptors from the same fragment will form an intramolecular loop during PCR, thereby reducing the efficiency of the PCR primers in binding to the adaptors and exponentially amplifying that same-ended fragment.

In a preferred embodiment, enriching for target DNA genome fragments with boundaries defined by two different predetermined DNA sequences is performed by directional amplification. "Directional amplification" as used herein intends to preferentially amplify and enrich for different-ended fragments, while minimizing the amplification of same-ended fragments. Note that same-ended refers to fragments that have the same predetermined sequences, such as RERS, at both sides (e.g. fragments that were digested by the same restriction enzyme at both sides, or fragments containing the same adaptor at both sides, or fragments that were amplified by primers binding to the same predetermined sequence, such as a RERS). Likewise, different-ended refers to fragments that have 2 different predetermined sequences, such as RERS, at both sides (e.g. fragments that were digested by a different restriction enzyme at both sides, fragments containing 2 different adaptors at both sides, or fragments that were amplified by primers binding to two different predetermined sequences, such as RERS).

Directional amplification can be achieved in several ways when using restriction enzyme digestion and ligation of adaptors, as explained below:
(1) The adaptor concentration can be decreased, in order to favour the intramolecular annealing of same-ended fragments (i.e. fragments that were digested by a particular restriction enzyme at both sides). Upon ligation, the looped construct (resulting from the ligation of the intramolecularly annealed same-ended fragments) will not contain any adaptor, and hence no primer binding site for subsequent amplification using PCR. It should be noted that adaptors preferentially carry a 3'dideoxynucleotide, in order to prevent adaptor-adaptor ligation.
(2) Identical adaptors flanking the same fragment can hybridize to each other during PCR, thereby forming a hairpin structure in which the stem is composed of the hybridized adapters and the loop is formed by the fragment lying in between the adapters. The presence of a hairpin structure makes such a fragment less likely to be amplified in a next PCR cycle. Different-ended fragments (i.e. fragments containing 2 different adaptors) will not form strong hairpin structures upon amplification, and hence will be preferentially amplified and enriched.
(3) A combination of both methods.

Directional amplification can be achieved in several ways when using a PCR-based amplification method, as exemplified by a method in which each of the primers contain a specific sequence that is designed to be able to form a strong hairpin structure, when a fragment contains the same sequence at both sides (i.e. when the fragment was amplified using the same primer annealing at both sides). The presence of a hairpin structure makes such a fragment less likely to be amplified in a next PCR cycle. When two different primers were used to amplify a fragment, there will be no formation of a strong hairpin structure, and hence such fragments will be preferentially amplified.

In certain embodiments, the target DNA is digested at the RERS. Preferably a combination of two restriction enzymes is used to generate well-defined DNA fragments. Double restriction enzyme digestion of the genome will generate 2 categories of fragments: fragments with identical palindromic parts of the restriction enzyme recognition site at each side of the fragment, and fragments with different palindromic parts of the restriction enzyme recognition site at each side of the fragment. The choice of enzymes will amongst others depend on their cutting frequency; the distribution of cleavage sites across the genome; and the resulting predicted fragment lengths. Restriction enzyme cleavage may produce blunt ends or overhanging ends and may produce fragments cut by one or the other restriction enzyme, or a combination thereof. In certain embodiments, T-tailed adaptors are added to the DNA fragments. Alternatively, suitable adaptors with compatible ending are added to the cleaved DNA fragments. Several types of adaptors have been described and include single-looped adaptors with overhanging end, hybrids of two oligos with one overhanging end, hybrids of two oligos with two overhanging ends, Y-shaped adaptors, single-stranded adapters, etc.... All of these types of adaptors are applicable in the methods of the present invention. RE-specific adaptors are ligated to the RE digested fragments to generate fragments with identical and different adaptors at each side. Once the adaptors are ligated to the fragment, a third restriction enzyme or more restriction enzymes may optionally be added for additional cleavage of the fragments. In a particular embodiment, single-stranded adapters (i.e. a single oligonucleotide that is not hybridized to an at least partially complementary oligonucleotide) are used to reduce potential interference between the adapters and the primers that are used during a subsequent PCR step. When a 5' (five prime) to 3' (three prime) single-stranded adapter is ligated to the fragment, its complementary strand can be synthesized using the 5' to 3' end-filling capabilities of the PCR enzyme. If the primers that are subsequently used in the PCR step are designed to be complementary to these newly generated complementary strands, the primers will not be able to anneal to the original single stranded adapters. This reduces the amplification of undesired adapter-adapter dimers and avoids the need to remove un-ligated adapters prior to the PCR step. In addition, it allows the addition of random regions in the 3' region of the single-stranded adapter, for which the exactly complementary sequence is then generated using the end-filling capabilities of the PCR enzyme. The introduction of these random regions upstream (i.e. more to the 5' side) of the invariable, predetermined sequence at the boundary of the fragment avoids the generation of low diversity libraries. Such low diversity libraries are more difficult to sequence on certain NGS platforms for which the cluster recognition algorithm requires significant diversity in the first few bases of the read (for example the HiSeq2000 and HiSeq2500 platform from Illumina).

"Palindromic sequence" as used herein, is a nucleic acid sequence (DNA or RNA) that is the same whether read 5' (five-prime) to 3' (three prime) on one strand or 5' to 3' on the complementary strand with which it forms a double helix. Many restriction endonucleases(restriction enzymes) recognize specific palindromic sequences and cut them. For instance, the restriction enzyme EcoR1 recognizes the (full) palindromic recognition sequence 5'-GAATTC-3'
3'-CTTAAG-5'
The top strand reads 5'-GAATTC-3', while the bottom strand reads 3'-CTTAAG-5'. After EcoR1 RE cutting, the palindromic parts of the restriction enzyme recognition site are
5'-G AATTC-3'
3'-CTTAA and G-5'
Note that "palindromic sequence" also refers to such a palindromic part of a RERS, from which the (full) palindromic RERS can be inferred.

As used herein, "Adaptor" or "Adapter" in genetic engineering is a short, chemically synthesized, at least partially double stranded oligonucleotide (DNA or RNA) molecule which can be linked to the end of another DNA molecule or fragment. A RE or RERS-specific adaptor is an adaptor with a palindromic part of a RERS (which can be partially single stranded) that can be ligated to another DNA molecule or fragment with a complementary palindromic part of a RERS. Adapters may incorporate more than one RERS. Hence, adaptors ligated to a DNA fragment may be subjected to a further RE digestion that cuts the adaptor at another RERS.

"Well-defined fragments" as used herein, are fragments having well-defined boundaries that can be located to specific sites in the target genome (i.e. the predetermined sequence, e.g. restriction enzyme recognition sites). In particular embodiments, well-defined fragments are generated via restriction enzyme digestion of the target genome, followed by ligation of restriction enzyme recognition site-specific adaptors, amplification via PCR and an optional size-selection step that can be accomplished in conjunction with a purification step. The fragments will contain the full RERS at fixed positions from the boundaries of the fragment. In other embodiments, no RE digestion is required and in such case the fragments are generated by targeted amplification using primers containing a predetermined sequence (e.g. RERS) amongst other sequences.

"Enriching" as used herein refers to a method to add or increase the proportion of a desired ingredient. For example, enriching specific target DNA fragments refers to a process that increases the proportion of said specific fragments over other DNA fragments that may be present, for example using preferential amplification of those specific fragments; by isolating or purifying those specific fragments; or by destroying or removing other DNA fragments.

Different approaches are possible for reducing the complexity of a genome. The methods of the present invention may for instance apply PCR to preferentially amplify (and, thus, enrich) fragments with different adaptors on each side. The PCR will require 2 primers, each primer binding to one adaptor. Preferably one or both primers will contain a sample-specific barcode that will enable pooling of different samples into a single NGS run. In certain embodiments, a target enrichment step is introduced. Suitable methods for enrichment are amongst others bead capture (e.g. SPRI beads, AMPure XP beads, SPRIselect beads), gel-based size selection (e.g. E-Gel™ SizeSelect™ Gels) or other methods (e.g. BluePippin) of the amplified fragments according to their length. In this manner a tractable subset of fragments of the genome is created for sequencing. Therefore, in a particular embodiment, the construction of the reduced representation library further comprises selecting a subset of fragments according to their fragment length. In a particular embodiment, fragments of a length of about 20 to about 5000 bp are selected, in particular 50-1000bp, even more in particular 50-500 bp. In another embodiment, fragments of about 150-500bp, 200-450 bp, 200-400bp, 250-400bp, 250-350 bp. In an alternative embodiment, fragments are selected wherein the inserts corresponding to the genomic DNA sequence are of the above length ranges.

Alternatively, the target DNA will not be cleaved and the reduction of the complexity of the genome will be obtained differently. In this particular embodiment, PCR primers are used that have a match site sequence at their 3' end. Due to the match site sequence at the 3' end, these primers will only hybridize to a region comprising a predetermined sequence that is complementary to the match site sequence. In a further preferred embodiment, these PCR primers comprise hybridization signals or a barcode at their 5' side, a degenerate sequence at the central part, and a match site sequence at their 3' end. These primers are used in an amplification process. In preferred embodiments, the match site sequence will be different in the forward and reverse primer. Using the described primers in an amplification (PCR) process will generate only segments that contain target sequences situated between the 2 match site sequences (i.e. between 2 predetermined sequences) and reduce the representation of the genome. The level of degeneration will largely determine the selectivity of the amplification. In addition, the length of the predetermined sequence greatly influences the amount of amplified sequences and, thus, the amount of representation reduction. In a preferred embodiment, the predetermined sequence length is about 2 to about 10 bases, in particular about 4 to 8 bases. Optionally, the process comprises a nested PCR to account for the complete presence of hybridization signals or barcode in the amplified fragment. The approach requires less input reagents, less manual steps, is cheaper and beneficial for single tube reactions.

The match site sequence will be composed of a sequence stretch that has a complementary sequence appearing on multiple positions in the target DNA (i.e. the predetermined sequence). In preferred embodiments the match site sequence will be a RERS sequence.
Thus preferred primers for use in the amplification process will contain hybridization signals or a barcode at their 5' side, a degenerate sequence at the central part, and a predetermined sequence, such as a RERS sequence, at their 3' end.

NGS applied on the described DNA fragments, all or not generated by restriction enzyme cutting, will generate non-overlapping segments of target DNA stretches with at least one segment boundary containing a predetermined sequence, such as a RERS, at a fixed position from that segment boundary. The assembly of said non-overlapping segments composes a reduced representation library of said target DNA genome.

Targeted reduction via predetermined sequences, such as a RERS, optionally supplemented with size selection, is in silico predictable and allows using a sparse reference genome for alignment and mapping. As all obtained reads should map to the sparse reference genome, the time needed for data analysis is reduced as compared to mapping to a non-reduced reference genome. Therefore, in a particular embodiment, the present invention comprises the use of a (non-reduced) reference genome. In a preferred embodiment, the present invention comprises the use of a sparse reference genome (wherein the sparse reference genome is an in silico predicted reduced genome as described herein). In addition, the use of predetermined sequences, such as RERS, facilitates alignment of the reads, as a defined region of every read (i.e. the predetermined sequence, such as a RERS) should map to a predetermined sequence, such as a RERS, in the sparse reference genome. Accordingly, with the use of predetermined sequences, such as RERS, the mapping and overall data analysis can be done in a more efficient way. The use of predetermined sequences allow for an in silico predictable specific amount of representation reduction. The amount of reduction can be increased or decreased by selecting particular predetermined sequences, changing the length of predetermined sequences, selecting particular combinations of predetermined sequences, and selecting particular lengths of fragments.

In a particular embodiment, the reduced representation library as used in the methods of the invention has been enriched for target DNA genome fragments that have two boundaries defined by predetermined DNA sequences. In particular, said fragments are located in the target DNA genome between predetermined DNA sequences. The fragments in the RRL may or may not comprise the predetermined DNA sequences. For example, when using Type IIS restriction enzymes (which cleave outside of their RERS), fragments will be generated that do not comprise the RERS itself, but the boundaries of the fragments are defined by the predetermined sequence (i.e. they are located at a specific distance of the predetermined sequence in the target genome). Furthermore, when using restriction enzymes that do cleave inside the RERS, after adaptor ligation, the RERS is not restored necessarily.
In a further particular embodiment, the fragments in the RRL comprise a genomic target sequence, a first flanking sequence at the 5' end of said genomic target sequence, and a second flanking sequence at the 3' end of said genomic target sequence;
wherein said genomic target sequence corresponds to a sequence in the target DNA genome that has two boundaries defined by predetermined DNA sequences. In a particular embodiment, each boundary is defined by a different predetermined DNA sequence.
In further embodiment, at least one of the first and second flaking sequences comprises a sequencing region. The sequencing region is adapted to allow sequencing of at least part of the genomic target sequence, in particular adapted to allow next generation sequencing (e.g. adapted to hybridize to a sequencing primer or capture probe).
In a preferred embodiment, at least one of the flanking sequences further comprises a barcode. Said barcode may be a sample-specific barcode that allows the pooling of samples before sequencing. In a particular embodiment, the barcode in the flanking sequence is introduced as part of the adapter. In another particular embodiment, the barcode in the flanking sequence is introduced by using an amplification primer that contains said barcode (and, consequently, the resulting amplicons contain said barcode).
In a particularly preferred embodiment, the first and second flanking sequences comprise a sequencing region and a barcode.

In certain clinical settings such as for instance in pre-implantation genetic diagnosis (PGD), pre-implantation genetic screening (PGS), or metastatic cancers, a major challenge consists of getting the DNA typing results starting from tiny amounts of target DNA derived from a few cells, in particular one, two, three, four, five, six, seven, nine, ten, between one and 50, between one and 100, between one and 1000, or between one and 10000 cells. Further, unless vitrification is applied to the embryos and the embryo is implanted in a next cycle, the genotype analysis may have to be performed within the time constraints of the in vitro fertilization (IVF) cycle. In cases with limited availability of the target DNA such as embryo biopsies, foetal cells or cell-free foetal DNA in the maternal peripheral blood circulation, or circulating tumour cells (CTCs) or cell-free circulating tumor DNA in cancers, the target DNA is first amplified to generate sufficient copies for downstream genotyping analysis (Coskun et al., 2007). Advantageously, and different from most prior art methods, the methods of the present invention allow to analyse a target DNA genome even when only a small amount of target DNA is available.

Thus, in one embodiment, the present methods include the step of amplifying the target genome by whole genome amplification or partial genome amplification. The amplified genome is analysed for genome modifications. Typically, the DNA from 1, 2, 3 to 10 cells, 1 to 50 cells, 1 to 100 cells, 1 to 1000 cells will be amplified. Preferred cells are one or more polar bodies, one or more blastomeres, cells from a trophectoderm biopsy, foetal cells or cell-free foetal DNA found in the maternal peripheral blood circulation, circulating tumour cells, or cell-free circulating tumour DNA. Different methods of whole genome amplification (WGA) have been described, including PCR and non-PCR methods of WGA (Zheng et al., 2011), and are well known in the art. A preferred method for whole genome amplification comprises multiple displacement amplification (MDA). Partial genome amplification preferably comprises the PCR method that amplifies fragments with boundaries defined by predetermined DNA sequences as described herein. Following amplification, amplified fragments can be submitted to further specific requirements of the methods of the present invention.

In a particular embodiment, the present invention provides methods for target DNA genome analysis, wherein only a low amount of target DNA genomic material is available. In particular, the RRL is constructed using only a low amount of target DNA genomic material. In a further embodiment, said target DNA genomic material is either present within one or a few target cells, or as free circulating material in the sample. Thus in a particular embodiment, said sample contains one or a few target cells. In a further embodiment, said sample contains one target cell. In another embodiment, said sample contains a few target cells, in particular 1 to 30, more in particular 1 to 20, target cells. For example, 1-15, 1-10, 1-8, 1-7, 1-6, 1-5, 1-4, 1-3, one or two target cells. In another particular embodiment, target nucleic acids are present in an amount of 2 ng or less in said sample, in particular 1 ng or less, more in particular 0.5 ng or less. In another particular embodiment, target nucleic acids are present in an amount of 250 pg or less in said sample; in particular 200 pg or less; more in particular 150 pg or less. In another particular embodiment, said target nucleic acids are present in an amount of 100 pg or less; in particular in an amount of 50 pg or less; more in particular in an amount of 30 pg or less. In another particular embodiment, said target nucleic acids are cell-free, circulating nucleic acids. For example, circulating cell-free fetal DNA from a maternal sample, or circulating tumor DNA from a patient sample. While genetic material (e.g. maternal DNA) may be abundant in such samples, target DNA (e.g. fetal DNA) is present in only very limited amounts. In a particular embodiment, target nucleic acids are present as cell-free nucleic acids in a fluid sample. In particular, said cell-free nucleic acids are present in a fluid sample comprising additional (non-target) nucleic acids. In a particular embodiment, said sample comprises a mixture of target and non-target nucleic acids. Preferably, said target nucleic acids are present in an amount between 0.1 and 80%, or more preferably between 0.1 and 20% of said non-target nucleic acids. In another particular embodiment, said sample comprises a mixture of target and non-target nucleic acids, wherein said target nucleic acids are present in an amount of 700 ng or less, in particular 500ng or less, more in particular 300 ng or less. In a further embodiment, 200 ng or less, in particular 100 ng or less, more in particular 50 ng or less. In yet another embodiment, said sample comprises cell-free nucleic acids, wherein said cell-free nucleic acids are present in an amount as defined hereinabove.

In a particular embodiment, the present disclosure relates to a method for target DNA genome analysis, comprising:
- obtaining a sample comprising a low amount of target DNA genomic material; and
- constructing a reduced representation library of said target DNA genomic material.

In a further embodiment, the method comprises:
- obtaining a sample comprising a low amount of target DNA genomic material;
- performing whole genome amplification of the target DNA genomic material; and
- constructing a reduced representation library of said target DNA genomic material.

The reduced representation library is subsequently used in the methods as described herein.

As evident from above, the present disclosure relates to methods that are also suitable for non-invasive prenatal diagnosis. In said method, free-floating fetal DNA present in maternal blood is analysed according to the invention. The reduced representation library can be constructed as described herein.

In a particular embodiment, the method further comprises a step for enriching fetal DNA (i.e. the target DNA genomic material). In another particular embodiment, the method may comprise a size selection step. More in particular, said size selection step selects for fragments having a genomic sequence insert of less than about 250 bp, in particular less than about 200 bp, more in particular less than about 150 bp. Evident from the remainder of the application, said fragments will correspond to target genomic regions wherein the predetermined sequences are located about 250 bp (or 200bp or 150 bp) or less from each other.

Preferably, due to the fraction of target DNA in total DNA in the maternal sample being about 1-20%, high coverage sequencing is used to sufficiently cover target DNA.

Thus, in a preferred embodiment, the present disclosure relates to a method for target DNA genome analysis, which method comprises the steps of:
- obtaining a fluid sample from a pregnant female, wherein the fluid sample comprises a low amount of target DNA genomic material;
- obtaining raw metrics for non-overlapping segments using a sequencing process applied on a reduced representation library of said target DNA genome,
   wherein said reduced representation library has been enriched for target DNA genome fragments having two boundaries defined by predetermined DNA sequences;
- clustering non-overlapping, nearby segments with similar raw metrics to provide master segments;
- providing metrics describing the master segments in which said metrics include inferred boundaries of one or more master segments, number of observed reads in one or more master segments, observed 4-base frequencies in said one or more master segments, or ancestral probability for one or more of said master segments.

A sequencing process is applied on the reduced representation library. Such a NGS run produces an image file which can be converted to a base-called FASTQ file using standard methods. In case multiple samples are involved, such FASTQ file may need to be demultiplexed and every read will be assigned to a sample according to the sample-specific barcode in the read. For every sample, the assigned reads are mapped onto a reference genome, thereby making advantage of the fact that well-defined positions of the reads (e.g. the position containing the restriction enzyme recognition site) should map to specific sites (e.g. the restriction enzyme recognition sites) in the reference genome.
In a preferred embodiment, the reference genome is the in silico simulation of the reduced library representation. This results in a set of segments to which reads are assigned, and these mapping data are stored in a BAM file. The mapping data in the BAM file can be further analyzed, and the sequencing process will thus produce raw metrics for each of the segments. Such raw metrics include base frequency, 4-base frequency, read count, normalized read count, ancestral probability, quality score for mapping, quality score for base-calling, or any metric derived thereof.

In the present disclosure, the term raw metrics also includes ADO. ADO can be deduced if a certain fragment or master segment in the target DNA is compared to the corresponding fragments or master segments in the DNA from related individuals (e.g. parents, grandparents, siblings, ...). If e.g. one parent is homozygous AA for a certain position and the other parent is homozygous CC for the same position, then it can be expected that a cell from an embryo derived from the oocyte of the one parent and the sperm cell from the other parent should be heterozygous AC for that position. If the sequencing would indicate that the majority of the reads covering that position carry an A allele, this position can be flagged as a position with ADO for the other parent. Such a raw metric may support the interpretation of the results obtained with the target sample: if the number of positions with ADO in the embryo cell is low and randomly spread across the genome, this may e.g. be caused by random WGA artefacts. If however the number of positions with ADO in the embryo cell is locally very high, e.g. for a certain chromosome, this may e.g. be indicative for a monosomy in which only the chromosome of the one parent is present.

In the present disclosure, the term raw metrics also includes ADI. ADI can be deduced if a certain fragment or master segment in the target DNA is compared to the corresponding fragments or master segments in the DNA from related individuals (e.g. parents, grandparents, siblings, ...). If e.g. one parent is homozygous AA for a certain position and the other parent is also homozygous AA for the same position, then it can be expected that a cell from an embryo derived from the oocyte of the one parent and the sperm cell from the other parent should be homozygous AA for that position. If the sequencing would indicate that a significant proportion of the reads covering that position carries e.g. a C allele, this position can be flagged as a position with ADI. Such a raw metric may support the interpretation of the results obtained with the target sample: if the number of positions with ADI in the embryo cell is high, this may e.g. be caused by DNA contamination or be indicative for a sample switch.

In the present disclosure, the term raw metrics also includes a parameter to describe the homozygosity of the fragment. The parameter describing the homozygosity of the fragment can be deduced from the sequencing data by looking at the observed base frequencies within that fragment. The higher the number of positions that have base frequencies reminiscent of a homozygous position, the higher the parameter describing the homozygosity of the fragment. Such a raw metric may support the interpretation of the result obtained with the target sample: if the fraction of fragments within a master segment with high homozygosity scores exceeds a certain threshold, this may be indicative for a master segment that displays so-called "Loss of heterozygosity" (which will also be evident from the base-frequency pattern that will display a base frequency pattern with frequencies at 0 and 1, and not at e.g. 0.33, 0.5 or 0.66). Such regions with Loss of heterozygosity can be indicative for a monosomy (with correspondingly reduced overall read count) or uniparental isodisomy (if the overall read count is not affected as compared to other, diploid master segments).

As used herein, base frequency includes the base frequency of one, two, or three bases, as well as 4-base frequency, unless specified otherwise. Furthermore, as used herein, read count refers to read count as well as normalized read count, unless specified otherwise. The present invention can evidently also be applied to NGS data where the initial 4-base frequency per position (as obtained after mapping of the reads to the reference genome) is converted to a 2-base frequency (which includes e.g. the so-called B-allele frequency that is referred to in the state of the art). The conversion may consist of e.g. retaining the 2 highest base frequencies per position, or e.g. only retaining the base-frequencies of bases that have previously been observed (this can be e.g. the bases that have been reported in databases such as dbSNP). As such, in the present invention, the term raw metrics may also include B-allele frequencies, 2-base frequencies or similarly, 3-base frequencies.

In particular, for each segment, the number of assigned reads is counted, giving an uncorrected number of reads per segment (read count). Correction methods may be applied in order to correct for positional influences. Reads may be corrected using positional info of the fragment (e.g. GC content), or corrected for centromere or telomere regions. Another correction factor may be based on the average counts for that particular segment in a historical dataset. Such corrections will generate a normalized read count per segment. For each position in the segment, the number of A, C, G, T is counted; the number of calls (sum of the number of A, C, G, T) is counted; the base frequencies (e.g. %A, %C, %G or %T per position) or 4-base frequencies (i.e. the observed % of any base per position, without specifying the exact base, e.g. 1%, 2%, 7% and 90%) are calculated. For every segment, the obtained base frequencies at the individual positions are collected. For every base having a base frequency in between certain thresholds (e.g. between 10 and 90%), the ancestral probabilities can be calculated. Any of the data as described are considered to be raw metrics.

Ancestral probabilities, as used herein, cover paternal probabilities, maternal probabilities and grandparental probabilities. As used herein, "paternal probability" is the probability that the base is inherited from the father, and "maternal probability" is the probability that the base is inherited from the mother, given the obtained "raw sequence read data" for the target, father and mother at the corresponding position in their genomes. And similar definition holds for the grand-parental probabilities.

The methods of the present disclosure will apply clustering. Non-overlapping, nearby segments with similar raw metrics or metrics derived thereof will be clustered to provide master segments. Segments are assembled into master segments using a segmentation model. Only segments that are consecutive or in relatively close proximity and on the same chromosome in the reference genome can be assembled into 1 master segment. In this context, proximity is based on the expected position in an in silico simulated reduced reference genome as well as position in a "full" reference genome. The latter also provides information related to the physical distance between segments (in terms of bases) and the expected occurrence of a chromosomal recombination event in between the two segments (typically expressed in centi-Morgan), both of which can be used as input metrics in the segmentation model. Consecutive segments that have similar raw sequence read data are likely to be assembled into 1 master segment. For instance, segment A having 99 reads, base frequencies that cluster close to 0, 50 and 100%, and a paternal probability that is higher than the maternal probability will likely be assembled with segment B having 100 reads and base frequencies that cluster close to 0, 50 and 100%, and a paternal probability that is higher than the maternal probability. Note that this does not exclude the chance that consecutive fragments may have contradictory raw sequence read data (e.g. fragment C having a very high paternal probability, and fragment D having a very low paternal probability) and are still clustered into 1 master segment, provided that their clustering is supported by a sufficient number of surrounding segments that have similar raw metrics and were therefore also assigned to the same master segment (for an example, see table 1 and its description). Contradictory raw sequence read data may be caused by artifacts during WGA, PGA or NGS, but the fact that multiple fragments are assembled into a master segment filters out the impact of such artifacts on the final, discrete call for the master segment.

In a preferred embodiment, clustering is based on raw metrics comprising read count and base frequency. In a further embodiment thereto, the method preferably further comprises making a DNA call regarding the presence or absence of aneuploidy.

In another preferred embodiment, clustering is based on raw metrics comprising read count, base frequency and ancestral probability. In a further embodiment thereto, the method preferably further comprises making a DNA call regarding the ancestral origin of a genome region.

When performing clustering on multiple raw metrics, it is to be understood that said clustering may comprise a single clustering step wherein the multiple raw metrics are used or, in the alternative, may comprise multiple clustering steps wherein in each step a selection of raw metrics is used. In a particular embodiment, the method of the present invention comprises a first clustering step based on read count and base frequency and a second clustering step based on ancestral probability. The method preferably comprises a further step of making a DNA call regarding the presence or absence of aneuploidy in a genomic region and the ancestral origin of said genomic region.

The present disclosure can also be applied to detect polyploidy in a sample, e.g. triploidy or tetraploidy in a human cell. Polyploidy will be evident from the integrated analysis of the raw metrics (e.g. observed base frequencies). Indeed, e.g. triploidy will be evident if most (if not all) of the master segments display a base frequency pattern with frequencies at 0, 0.33, 0.66 and 1. It should be noted that polyploidy can typically not be detected when working with e.g. array-CGH. The present invention can also be applied to detect triploidy, tetraploidy, polyploidy, monoploidy, regions with loss of heterozygosity (LOH), uniparental disomy, uniparental isodisomy, uniparental heterodisomy.

With "Clustering" or "Assembling" is meant, grouping a set of objects in such a way that objects in the same group (called a cluster) are more similar (in some sense or another) to each other than to those in other groups (clusters). It is a main task of exploratory data mining, and a common technique for statistical data analysis, used in many fields including bioinformatics.

The term "fragment" refers to a part of a nucleic acid. Likewise, the term "segment" refers to a part of a nucleic acid sequence.

A segmentation model or cluster model is defined as a computational model that aims to identify master segments of the genome for which the underlying segments display a similar profile for specific metrics. In these models, the boundaries of these master segments are typically referred to as change-points. Segmentation models can be applied for the reconstruction of a target genome.

Many different types of segmentation models have been described in the field of DNA typing. Specifically for the analysis of NGS data, segmentation models are most typically applied for the identification of CNVs.

"Typing" as used herein, refers to characterizing the target DNA genome.
The characterization may relate to the global genome structure of the target DNA genome (cf. chromosomal and subchromosomal structures), as well as the detailed molecular structure of the target genome (cf. small polymorphisms in a a gene or intergenic region or non-coding region).
The characterization may relate to inherited (cf. an inherited genetic or chromosomal aberration) or de novo aspects (cf. meiotic CNVs in the gamete or embryo, or de novo (sub)chromosomal aberrations involved in tumorigenesis). The characterization may relate to the description of Copy Number Variations (CNVs) of (sub)chromosomal regions or polymorphisms at specific positions (such as insertions, deletions or single nucleotide polymorphisms). In some instances, typing may be referred to as genotyping, haplotyping or aneuploidy detection.

The strategies used in these NGS segmentation models can be classified as Depth Of Coverage (DOC)-based methods, Paired-End Mapping (PEM)-based methods, Split-Read (SR)-based methods, ASsembly (AS)-based methods, or a combination of the afore mentioned methods.

There is a large number of different statistical algorithms that can be applied in these segmentation models, including (but not limited to) Circular Binary Segmentation (CBS), Event-Wise Testing (EWT), Mean Shift-Based (MSB), Maximum Likelihood Estimation or Expectation Maximization (EM), Lowess, Wavelet based methods such as Discrete Wavelet Transform (DWT), Hidden Markov Model (HMM), Rank segmentation, Moving Window, Recursive Segmentation, Bayesian approaches, Walking Markov, Change-point methods, Regression, Shifting Level models, Mixture models, Piece-Wise Constant Fitting and Pairwise Gaussian Merging.

Software tools developed for CNV detection in NGS data vary in terms of strategy (cf. supra), statistical algorithm (cf. supra), window-size (fixed or variable or not applicable), reference (referenced within the sample, or referenced using an external control, or not applicable) and clustering output (hard or soft/fuzzy). Specific examples of such software tools include (but are not limited to) CNV-seq, Seqseg, RDXplorer, cn.MOPS, BIC-seq, CNAseg, seqCBS, JointSLM, rSW-seq, CNVnorm, CMDS, mrCaNaVar, CNVeM, cnvHMM, CNVnator, FREEC, ReadDepth, Varscan, CNV-TV, PEMer, Variation Hunter, HyDRa, SVM2, MoGUL, BreakDancer, CLEVER, Spanner, commonLAW, GASV, Mosaik, AGE, SLOPE, SRiC, Pindel, ClipCrop, Cortex assembler, Magnolya, TIGRA-SV, SOAPdenovo, Velvet, ABySS, CNVer, cnvHiTSeq, Genome STRIP, SVDetect, NovelSeq, GASVPro, inGAP-SV, SVseq, Zinfandel, CoNIFER, ExonCNV, MoDIL, MrFast.

Following clustering, the target DNA (or each chromosome) will be represented by a number of master segments and each master segment will be characterized by metrics including inferred boundaries; number of observed reads, observed base frequencies, or ancestral probability. This master segment information and its associated metrics will be used for making the final, discrete DNA call in the analysis. In the present invention, the metrics describing the master segments may also include e.g. inferred copy number estimates for one or more master segment, a value representing the overall homozygosity or other summarizing statistics describing the one or more master segments.

In contrast with the present methods that make predictions about typing or ancestral origin based on the clustering of segments, most existing methods summarise the sequence data into discrete base-calls, discrete polymorphism calls and/or discrete parental information calls for individual locations (e.g. loci, polymorphisms). However, the influence of an artifact may be such that it leads to wrong discrete calls.
In contrast, the described method does not make discrete calls on individual locations, thereby maintaining both the correct and artifact information, and using pattern recognition to identify a consensus call for an assembly of consecutive segments (i.e. the master segment).
This is exemplified by methods that use a discrete allele call (eg at a certain position, there is a certain nucleotide in the first allele and a certain nucleotide in the second allele), which methods often assume that the location is diploid. In particular, the methods of the present invention are not critically dependent on discrete allele calls, but rather rely on base frequencies (i.e. at a certain position, X% of the observations were nucleotide A, X% of the observations were nucleotide C, etc). Further, in a particular embodiment, the described method does not make a discrete ploidy call before clustering, but rather retains the (corrected) number of observed reads. Also in terms of ancestral origin, typical methods assign an ancestral origin (i.e. father, mother, or grandparent) to observed polymorphisms, while the described method merely assigns an ancestral probability to an observed base. By summarizing measurements into discrete calls based on the obtained data for a single location, and not making a discrete call for that location based on information obtained from multiple locations in a surrounding regions (assigned to the same master segment), there is more impact of artifacts on that discrete call. By not summarizing measurements into discrete calls, more experimental information is retained for each of the segments, which can afterwards be used in the segmentation model to make a more reliable final discrete DNA call for all of the segments assigned to a master segment. Note that some methods filter noise by assuming that the noise signal will be less pronounced than the true signal. This assumption is not always true, as exemplified by the occurrence of ADI in methods relying on discrete calls and such type of noise filtering. By not making a discrete allele call based on obtained data for a single location, but instead retaining the raw metrics such as observed base frequencies, such type of artifacts are filtered out across the master segment. Advantageously, the present invention analyzes only a part of the target DNA genome (by using a RRL), but that part is analyzed using the high information content available through sequencing (i.e. without making discrete genotype and/or ploidy calls before clustering). As such, the method of the present invention provides high quality clustering with more reliable calls, while still being cost-effective. The retention of the high information content of sequencing is especially important for samples that contain a low amount of target DNA genomic material. Due to the low amount of genetic material, the sequencing results will contain a high amount of noise (e.g. allele drop-out and allele drop-in resulting from genome amplification and sequencing errors). Prior art methods in general discard sequence reads comprising such high levels of noise, thereby loosing potentially valuable information and reducing reliability.

The discrete DNA call for the master segment, made with use of the methods of the present invention will largely depend on the requested analysis. A number of cases are exemplified in the example section. As shown in the example section, the discrete DNA call in the methods of the invention may for instance relate to e.g. the ancestral call (e.g. is the master segment paternal or maternal, grandpaternal or grandmaternal for a specific parent) or a CNV call (e.g. is the master segment present in 1 or 2 copies in the target genome, cf. (sub)chromosomal aneuploidy calling). For each of these parameters, a summary (i.e. final discrete call) is made based on the underlying raw metrics for each of the segments assigned to the master segment. The summary for CNV call may rely on calculating the average read count of all segments assigned to the master segment and calculating the probability that this corresponds to a master segment present in e.g. 0, 1, 2 or 3 copies. The summary for parental call may rely on calculating the likelihood that a certain master segment has a certain parental origin based on the parental probabilities of the underlying segments. The summary for grandparental call may rely on calculating the likelihood that a certain parental master segment has a certain grandparental origin based on the grandparental probabilities of the underlying segments.

The assembly into segments results in a band pattern of base frequencies across the segment (i.e. base frequencies cluster together in particular bands). This allows identifying
- monosomy (regions that have a base frequency band pattern of 0 and 100%, and an average read count that is about 50% lower than expected for a diploid region).
- uniparental disomy (regions that have a base frequency band pattern of 0 and 100%, and an average read count that is about the same as expected for a diploid region)
- "disomy" (i.e. diploid, normal) (regions that have a base frequency band pattern of 0, 50% and 100%, and an average read count that is about the same as expected for a diploid region)
- trisomy (regions that have a base frequency band pattern of 0, 33, 66 and 100%, and an average read count that is about 50% higher than expected for a diploid region)
- tetrasomy (regions that have a base frequency band pattern of 0, 25, 50, 75, 100%, and an average read count that is about 100% higher than expected for a diploid region)
- note that if ancestral information is available, this can allow to further refine the DNA typing analysis, e.g. by specifying that a certain master segment displays maternal monosomy (if the maternal probability for the corresponding master segment is high), or a unipaternal disomy (if the master segment is present in 2 copies and the paternal probability for the master segment is high).
- meiosis I origin or a meiosis II origin of a CNV.

Thus, typically, the final discrete DNA calls will be linked to the required analysis.
In one embodiment, the analysis and final discrete call for the master segment involves probability-based identification of the presence of risk alleles for inherited disorders such as autosomal dominant or recessive disorders, X or Y-linked dominant or recessive disorders.
In one embodiment, the analysis and final discrete call for the master segment identifies disorders based on other pedigree members (parental siblings, siblings, ...) or identifies chromosomal recombination sites using siblings or embryos or gametes.
In one embodiment, the analysis and final discrete call for the master segment identifies the origin of chromosomal aberrations (such as non-disjunction errors in meiosis I or meiosis II), or identifies balanced structural chromosome abnormalities (such as inversions and balanced translocations).
In other embodiments, the analysis and final discrete call for the master segment covers epigenomic profiling of circulating tumour cells (CTCs), isolated CTCs, exosomes, circulating tumor DNA in body fluids (such as urine, blood, saliva, cerebrospinal fluid), circulating foetal cells or free foetal DNA in blood, biopsy material from a preimplantation embryo, tumor cells present in a biopsy tissue sample, or isolated from a tissue slice (Fresh Frozen Tissue or Formalin-Fixed Paraffin-Embedded Tissue), biopsy material from a foetus, new born child, or from an any subject (cf. children, parents, grandparents, horse, cow, pig, .....
In other embodiments, the analysis and final discrete call for the master segment concerns mosaicisms, such as the representativeness of a blastomere for the other cells of the embryo, subchromosomal CNV mosaicism in trophectoderm biopsy containing a few cells, identification of both chromosomal as well as subchromosomal mosaic CNVs, identification of mosaic CNVs in any mixture of cells (e.g. trophectoderm biopsy, CTCs, cancer cells, tumor tissue cells, mixtures of healthy and affected cells, ...) containing at least 2 cells, identification of CNVs in foetal cells or cell-free foetal DNA present in maternal blood, identification of foetal CNV mosaicism in a mixture of circulating foetal cells or foetal DNA and maternal DNA in which there is a twin pregnancy, identification of the presence of risk alleles related to inheritable disorders in the foetus or foetuses, identification of the presence of inversions, balanced translocations, unbalanced translocations, subchromosomal CNVs, chromosomal CNVs, identification of CNV mosaicism in circulating tumor DNA present in blood, analysis of exosomes present in blood, and exosomes isolated from blood, analysis of cell-free tumor DNA in other body fluids (saliva, cerebrospinal fluid, urine, serum). Further analysis and final discrete call for the master segment includes human leucocyte antigen (HLA) matching, noise typing to support analysis of the target genome or noise typing to identify a sample switch.

The application of segmentation models on genomic DNA sequence data obtained from NGS is uncommon:
- For individual samples, it is merely applied to identify segments with a CNV as compared to the reference genome by applying the segmentation model on uncorrected read counts, but these models do not use 4 base frequencies, quality metrics related to base-calling or mapping nor ancestral probabilities as data input for the segmentation model(Rigaill et al., 2010).
- For population studies, segmentation models are applied on discrete SNP calls for each of the studied individuals, but these models do not use 4 base frequencies, quality metrics related to base-calling or mapping nor ancestral probabilities as data input for the segmentation model (Zhang et al., 2013)
- The application of segmentation models using a combination of observed (corrected) read counts, base frequencies, quality metrics related to base-calling or mapping and optionally also ancestral probabilities obtained via NGS has not been described.
- The application of segmentation models to genomic DNA sequence data obtained from NGS in a preimplantation context has not been described.
- The application of segmentation models using a combination of observed (corrected) read counts, base frequencies, quality metrics related to base-calling or mapping and optionally also ancestral probabilities obtained via NGS in a preimplantation context has not been described.

In a particular embodiment, the present disclosure thus provides a method of target DNA genome analysis, which method involves preimplantation genetic screening, preimplantation genetic diagnosis, cancer screening, cancer diagnosis, cell typing, or ancestral origin identification, and which method comprises any or all of the steps of:
- obtaining cell free foetal target DNA in the maternal peripheral blood circulation or cell free tumour DNA found in the peripheral blood circulation
- applying whole or partial genome target DNA genome amplification on said target DNA;
- applying next generation sequencing on a reduced representation library of said target DNA genome, which reduced representation library is composed of target DNA fragments with fragment boundaries defined by the presence of particular restriction enzyme recognition sites;
- obtaining non-overlapping segments of target DNA stretches with segment boundaries defined by the presence of particular restriction enzyme recognition sites, whereby the assembly of said non-overlapping segments compose a reduced representation library of said target DNA genome;
- obtaining for said segments, raw metrics from a sequencing process applied on said reduced representation library, which raw metrics include base frequency, 4-base frequency, read count, normalized read count, ancestral probability, quality score for mapping, quality score for base-calling, or any metric derived thereof;
- clustering non-overlapping, nearby segments with similar raw metrics to provide master segments, whereby said clustering uses a reference genome, pedigree information or is ancestral probability-based and derived from pedigree information;
- providing metrics describing the master segments in which said metrics include inferred boundaries of one or more master segments; number of observed reads in one or more master segments, observed 4-base frequencies in said one or more master segments, or ancestral probability for one or more of said master segments.
- making a final discrete DNA call based on the clustering of segments into master segments, wherein said call involves probability-based identification of: chromosomal recombination sites, (sub)chromosomal copy number variations, deletions, unbalanced translocations, amplifications, the presence of risk alleles for inherited disorders, non-disjunction errors in meiosis I or meiosis II, balanced structural chromosome abnormalities; epigenomic profiles of cells, mosaicisms, inversions, balanced translocations, human leucocyte antigen (HLA) matches, or occurrence of noice.

In a particular embodiment, the present disclosure relates to a method of target DNA genome analysis, which method involves preimplantation genetic screening, preimplantation genetic diagnosis, cancer screening, cancer diagnosis, cell typing, or ancestral origin identification, and which method comprises any or all of the steps of:
- obtaining liberated target DNA or liberate the target DNA from cells, which cells are chosen from one or two blastomeres, one to ten cells from tropHectoderm biopsy, one or two polar bodies, foetal cells, or exosomes found in the peripheral blood circulation, or circulating tumour cells;
- applying whole or partial genome target DNA genome amplification on said target DNA;
- applying next generation sequencing on a reduced representation library of said target DNA genome, which reduced representation library is composed of target DNA fragments with fragment boundaries defined by the presence of particular restriction enzyme recognition sites;
- obtaining non-overlapping segments of target DNA stretches with segment boundaries defined by the presence of particular restriction enzyme recognition sites, whereby the assembly of said non-overlapping segments compose a reduced representation library of said target DNA genome;
- obtaining for said segments, raw metrics from a sequencing process applied on said reduced representation library, which raw metrics include base frequency, 4-base frequency, read count, normalized read count, ancestral probability, quality score for mapping, quality score for base-calling, or any metric derived thereof;
- clustering non-overlapping, nearby segments with similar raw metrics to provide master segments, whereby said clustering uses a reference genome, pedigree information or is ancestral probability-based and derived from pedigree information;
- providing metrics describing the master segments in which said metrics include inferred boundaries of one or more master segments; number of observed reads in one or more master segments, observed 4-base frequencies in said one or more master segments, or ancestral probability for one or more of said master segments.
- making a final discrete DNA call based on the clustering of segments into master segments, wherein said call involves probability-based identification of: chromosomal recombination sites, (sub)chromosomal copy number variations, deletions, unbalanced translocations, amplifications, the presence of risk alleles for inherited disorders, non-disjunction errors in meiosis I or meiosis II, balanced structural chromosome abnormalities; epigenomic profiles of cells, mosaicisms, inversions, balanced translocations, human leucocyte antigen (HLA) matches, or occurrence of noice.

Throughout the present application, various embodiment are described regarding the reduced representation library, the sequencing of the reduced representation library and the clustering of segments. It is to be noted that the present invention also envisages the combination of any of these particular embodiments. For example, if a particular embodiment describes the preparation or use of a reduced representation library, the present invention also provides an embodiment towards such a method comprising the preparation or use of a reduced representation library according to any other particular embodiment described herein.

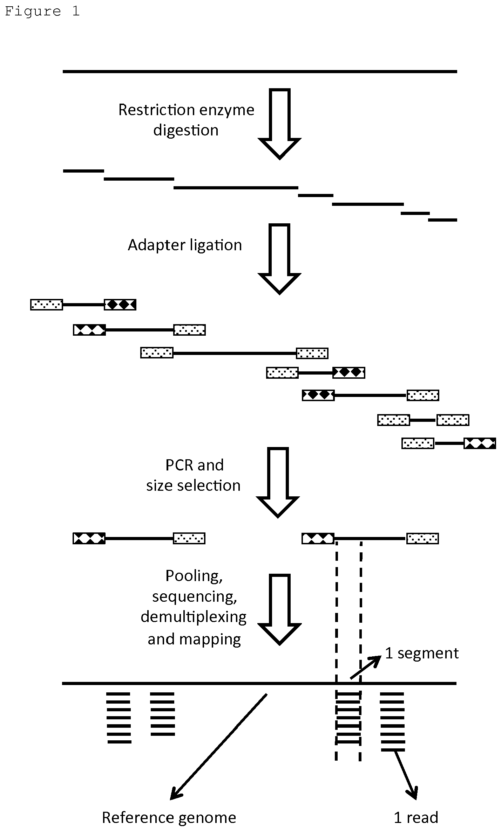

With specific reference to the figures, fig. 1 provides an overview of a preferred embodiment wherein genomic DNA is digested using two restriction enzymes that cut at different RERS (i.e. different predetermined sequences). In this example, two different adapters are used (a first adapter indicated with dots and a second adapter indicated with diamonds) for ligation to the two different ends of the digested DNA. PCR is used to enrich those fragments that contain two different adapters (i.e. different-ended fragments). Furthermore, a size selection step is performed (this can be integrated into the PCR step or separately performed before or after the PCR). The resulting reduced representation library has been enriched for fragments with two boundaries defined by a predetermined sequence (RERS) and a particular length. Sequencing generates reads which are mapped to particular segments on the reference genome. Compared to the target genome, the segments are located at a particular location in relation to the predetermined sequences (RERS). In this example, paired-end sequencing is used to generate reads for two non-overlapping segments located at each end of the fragment.

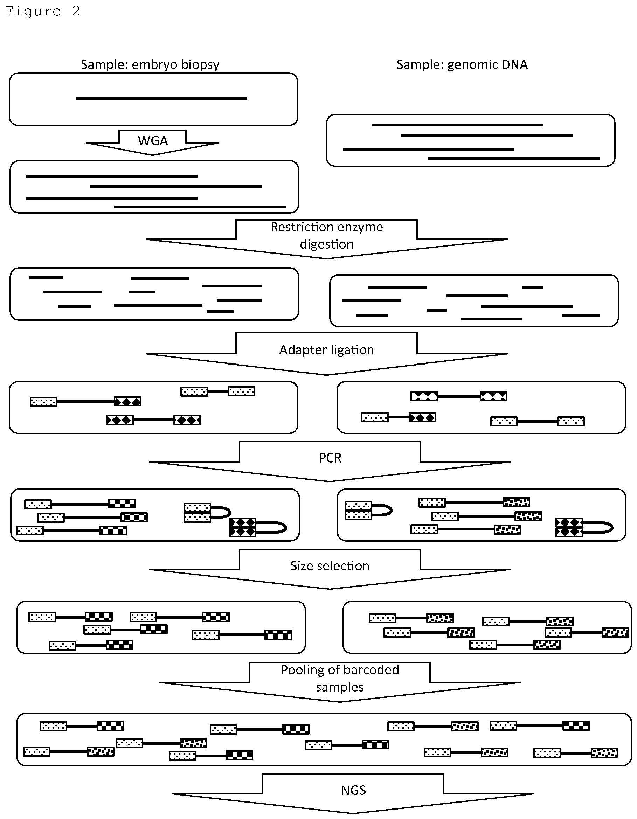

Figure 2 provides an overview of a preferred method for RRL construction and sequencing. Whole genome amplification is performed on genomic DNA derived from an embryo biopsy. A second sample, e.g. derived from a tissue biopsy from a parent, is used without further genome amplification. Both samples undergo restriction digestion with two restriction enzymes that recognize a different RERS. In each sample, two different adapters are ligated to the restriction digest: The first adapter is indicated with dots, the second adapter is indicated with large diamonds. Using sample-specific barcoded primers, at least one of the adapters (in this example the second adapter) is modified during the PCR step to include a sample-specific barcode. This is depicted as the second adapter of the embryo-related sample that is indicated with large squares, and indicated with small squares for the second sample. This PCR step relies on directional amplification, and the fragments with different adapters at each side are preferentially enriched. An optional size selection step can be performed, thereby generating two reduced representation libraries. The libraries are pooled and sequenced using NGS.

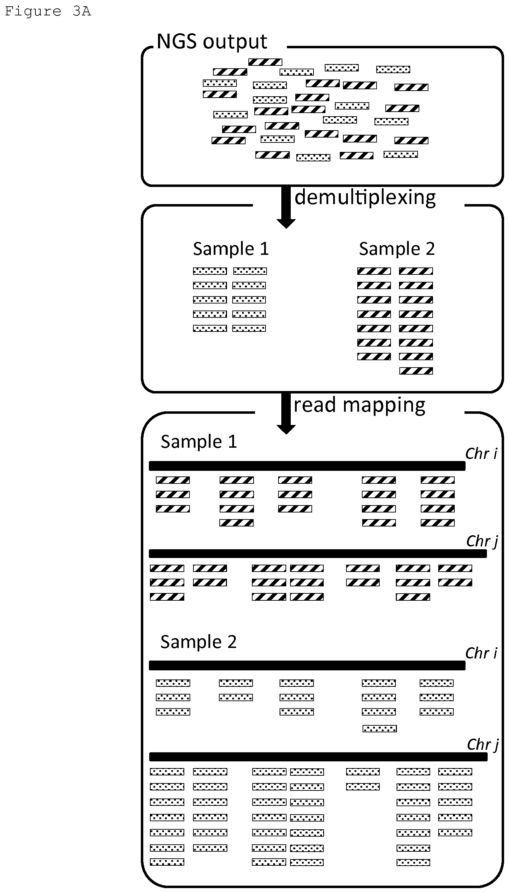

Fig. 3A provides an overview of the processing of NGS reads. In this case, the NGS data contain reads from two different samples. The sample-specific barcodes allow demultiplexing of the reads corresponding to the two different samples. Reads of each sample are the mapped to a reference genome, here represented using two chromosomes (*Chr i* and *Chr j*).

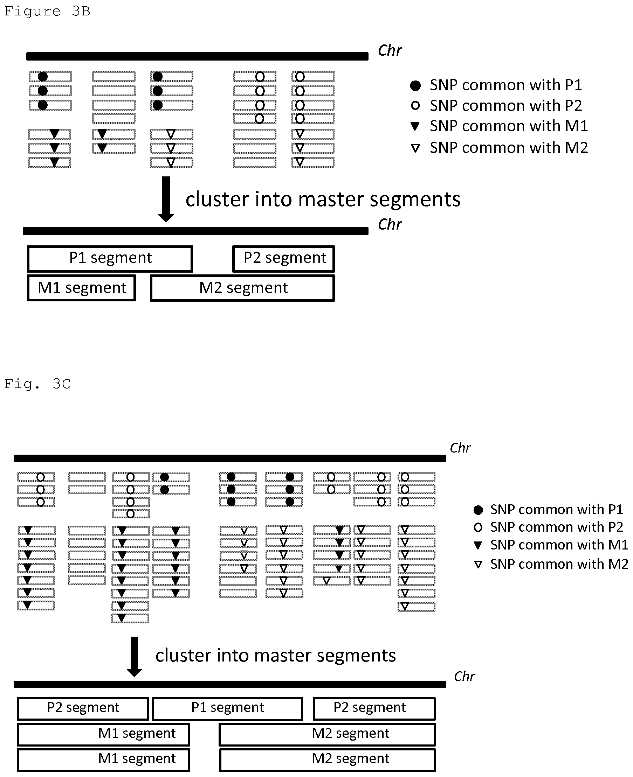

Fig. 3B and 3C show a clustering method according to the invention. In the figures, reads have been mapped to different segments on the reference genome. The number of reads that are assigned to each segment are "digital" (i.e. absolute numbers, e.g. between 6 and 12 reads in these examples). SNPs have been identified in the reads, and for each SNP the highest parental probability was determined (e.g "SNP common with P1" indicates that this SNP is most likely to be derived from the P1). Segments with a similar read count and ancestral origin are clustered into master segments. For segments for which the highest ancestral probability was not high, the ancestral origin can be given less weight in the cluster model, while the read count of that segment should not necessarily be given less weight in the cluster model. Note that also segments that do not contain SNPs can also be clustered into master segments, thereby also being assigned to a certain ancestral origin. Also segments that contain contradicting read counts or ancestral origin can be clustered into the master segment. P1 and P2 refer to the first and second paternal chromosome; M1 and M2 refer to the first and second maternal chromosome.

In the present disclosure, ancestral probability can also be deduced from working with a reference child that was conceived by the same parents as the embryo from which the target cell was isolated. Indeed, if a reference child is homozygous AA for a certain position, and the father is heterozygous AC and the mother homozygous AA, it can be logically expected that the reference child inherited one A from the father and one A from the mother. We can arbitrary define that this A from the father comes from one particular paternal chromosome. If the corresponding position in the corresponding master segment from the target cell would be heterozygous AC, it can be expected that the target cell inherited the C from the father. If this is the case for a significant number of neighbouring positions, it can be concluded that the target cell inherited a DNA segment from the other paternal chromosome. As the first paternal chromosome was inherited from a first parent of the father, and the other paternal chromosome was inherited from the other parent of the father, it should be clear from this description that such an ancestral probability of the master segments in the target cell can also be deduced by working with a reference child, even in the absence of DNA genotyping information from the parents of the parent.

Similarly, table 1 provides a summarized overview of a method of the invention. Per segment (Seg.), raw sequencing metrics for one particular position are shown for the target (embryo) sample, as well as the corresponding parental data for that position. The raw metrics are read count, 4-base frequency and highest parental probability. The read counts are similar for all shown segments (around 50), except for segment 4. The 4-base frequencies for all shown segments cluster around 0%, 50% en 100%. Based on read count and 4-base frequencies, this genome region is determined to be most likely diploid. The paternal contribution was determined for the genome region corresponding to segment 2 to segment 12, and is most likely entirely derived from P2. The maternal contribution was determined for the genome region corresponding to segment 1 to segment 11 and is most likely the result from a recombination event between segment 6 and segment 7. Values that are indicated in underlined bold (read count for segment 4 and highest parental probability for segment 9) are contradicting with their corresponding master segment and are most probably caused by artifacts.

**Table 1**

| Target sample | Raw metric | Seg. 1 | Seg. 2 | Seg. 3 | Seg. 4 | Seg. 5 | Seg. 6 | Seg. 7 | Seg. 8 | Seg. 9 | Seg. 10 | Seg. 11 | Seg. 12 |
|---|---|---|---|---|---|---|---|---|---|---|---|---|---|
| Embryo | Read count | 50 | 48 | 45 | **75** | 55 | 50 | 40 | 51 | 60 | 50 | 46 | 51 |
| Embryo | Frequency A | 45% | 53% | 60% | 1% | 45% | 1% | 2% | 43% | 40% | 53% | 3% | 48% |
| Embryo | Frequency T | 5% | 3% | 35% | 97% | 47% | 1% | 1% | 2% | 4% | 2% | 45% | 2% |
| Embryo | Frequency C | 3% | 4% | 3% | 1% | 3% | 58% | 40% | 53% | 40% | 44% | 2% | 2% |
| Embryo | Frequency G | 47% | 40% | 2% | 1% | 5% | 40% | 57% | 2% | 6% | 1% | 50% | 48% |
| Embryo | Highest parental probability | M1 | P2 | M1 | | P2 | M1 | M2 | P2 | **P1** | P2 | M2 | P2 |
| Supporting sample | Metric | | | | | | | | | | | | |
| Father | Genotype | GG | AG | TT | TT | AT | GG | CC | AC | AC | AC | TT | AG |
| Father | Phased genotype (P1/P2) | G/G | G/A | T/T | T/T | A/T | G/G | C/C | A/C | A/C | A/C | T/T | G/A |
| Mother | Genotype | AG | GG | AT | TT | AA | GC | GC | AA | CC | AA | GT | GG |
| Mother | Genotype (M1/M2) | A/G | G/G | A/T | T/T | A/A | C/G | C/G | A/A | C/C | A/A | T/G | G/G |
| Clustering | | | P2 master segment | | | | | | | | | | |
| | | M1 master segment | | | | | | M2 master segment | | | | | |

Further specific applications based on the described methods are detailed in the example section.

### References

Coskun U, et al. (2007) Whole genome amplification from a single cell: a new era for preimplantation genetic diagnosis. Prenat Diagn. 2007 Apr;27(4):297-302.
Dedonato M. et al. (2013) Genotyping-by-sequencing (GBS): a novel, efficient and cost-effective genotyping method for cattle using next-generation sequencing. PLoS One. May Vol. 8(5): e62137.
Elshire RJ, et al. (2011) A robust, simple genotyping-by-sequencing (GBS) approach for high diversity species. PLoS One May Vol. 6 (5): e19379
Gore MA, et al. (2009) A first-generation haplotype map of maize. Science 326: 1115-1117.
Peterson BK, et al. (2012) Double digest RADseq: An inexpensive method for de novo SNP discovery and genotyping in model and non-model species.
Rigaill G An Exact Algorithm for the Segmentation of NGS Profiles using Compression http://www.cs.umb.edu/~rvetro/vetroBioComp/compression/abstract-016.pdf
Zhang Y et al. (2013) De novo inference of stratification and local admixture in sequencing studies. Bioinformatics Vol. 14 (Suppl 5); S17.
Zengh C et al. (2013) Determination of genomic copy number alteration emphasizing a restriction site-based strategy of genome re-sequencing. Bioinformatics Vol.29 No.22: 2813-2821.

### Examples

### Example 1: RRL preparation, NGS and sequence mapping

WGA was applied on the embryo biopsy DNA using MDA. The MDA enzyme has proofreading activity, but due to the fact that there are only a few copies (i.e. 1 or 2 for a single blastomere) of the genome, there is a high chance for e.g. Allele Drop Out (ADO) randomly across the genome. Likewise there is a chance for e.g. Allele Drop In (ADI) across the genome.
Double restriction enzyme digestion was applied on the amplified genome to generate fragments with identical and different palindromic parts of the restriction enzyme recognition site recognition sites at each side. RE-specific adaptors were ligated to the fragments, to generate fragments with identical and different adaptors at each side. PCR was applied to preferentially amplify fragments with different adaptors on each side, as this is preferred for optimal use of the NGS capacity. The PCR requires only 2 primers. As the number of primers is very small, this greatly facilitates Quality Control (QC) during production of the oligonucleotides (as there are less primers, as opposed to e.g. array CGH, SNP arrays or generation of a reduced representation library via exome capture) and minimizes the chance for primer-primer interactions (which could lead to a disturbed PCR efficiency, as may occur during multiplex PCR reactions as in generation of a reduced representation library via exome amplification). At least 1 primer contains a sample-specific barcode that will enable pooling of different samples into 1 NGS run. As the primers contain the barcodes (as opposed to methods in which the barcodes are located in the adaptor), this allows all pre-PCR steps to be generic for every sample and every NGS platform, as the platform-specific barcodes (and platform-specific hybridization/sequencing signals) can be easily modified in the 5' tail of the primers. SPRI beads are used to purify the resulting DNA, and to selectively purify fragments that have a specific size. The use of SPRI beads as opposed to gel extraction for size selection allows batch processing (automation) and has a shorter turn-around-time. The use of SPRI beads as opposed to column extraction allows to accurately select fragments with a specific size (which is not possible using column extraction methods). The NGS run is performed according to the manufacturer's instructions.

The NGS image file is converted to a FASTQ file according to standard methods. The data in the FASTQ file are demultiplexed: every read is assigned to a certain sample, according to the sample-specific barcode in the read. This is done using standard methods. For every sample, the assigned reads are mapped onto a reference genome. The reference genome is the in silico simulation of the reduced library representation, and has a size that is at least 1 order of magnitude smaller than the "original" target genome sequence, and therefore the mapping is several orders of magnitude faster than other methods. In addition, the in silico reference genome is an assembly of segments that carry specific RERS at their boundaries, and for which an adjacent RERS is within a specific distance of the former RERS in the "full-size" reference genome (ie. the non-reduced genome). The mapping occurs in an efficient way, as e.g. position 40-45 (i.e. the RERS) of every read should be mapped to the RERS in the boundary of the segment, thereby reducing the degrees of freedom for mapping, and increasing the speed of the mapping process. This results in a set of segments to which reads are assigned, and these mapping data are stored in a BAM file.

### Example 2: Raw metrics characterizing the segments

For each segment of the reduced representation library, the NGS data are integrated into a summarizing dataset. This dataset contains positional information of the segment, base frequency, 4-base frequency, read count, normalized read count, ancestral probability, quality score for mapping, quality score for base-calling, and/or any metric derived thereof. These metrics are used for clustering non-overlapping, nearby segments with similar raw metrics to provide master segments. These master segments are characterized by metrics derived from the raw metrics.

### Example 3: Screening for subchromosomal CNVs in a preimplantation embryo in less than 24h.

In certain cases it is important to screen the DNA of a preimplantation embryo for subchromosomal CNVs and to have the diagnostic result available in less than 24h to enable transfer of the embryo within the same cycle. In such case, the next steps are set out below.

For every segment, the number of reads is counted. The number of reads is corrected according to the positional information of that segment: using a historical dataset on "normal" samples, the systematic artifacts introduced by e.g. WGA, PGA and/or NGS on the read count of every segment can be identified and corrected for. Corrected read count provides important information to identify regions with CNVs (which will have a deviating read count as compared to "normal" regions). However, a definitive call for a CNV should not be made based on 1 segment alone, as the result in that 1 segment may be perturbed by an artifact. Read count is independent from whether or not the segment contains a variant, and hence any segment provides usable read count information. This is not the case for SNP arrays, in which only positions in the genome that contain a SNP can be used.

For every position in the segment, the frequency of each of the 4 bases is calculated, and for every segment, the observed base frequencies for the 4 bases are assembled. These 4-base frequencies provide important information to identify regions with CNVs (e.g. a triploid region may have base frequencies close to 33 and 66%, and a tetraploid region may have base frequencies close to 25, 50 and/or 75%, and monoploid region will only have base frequencies close to 0 or 100%). However, a definitive call for a CNV can and should not be made on the base frequencies in 1 single segment, as it is essentially dependent on the presence of a variant in that single segment and only a consecutive assembly of different segments may contain sufficient base frequencies close to e.g. 33 and 66% to reliable call a CNV without being influenced by artifacts. In addition 4-base frequencies and read counts can be combined to further improve the reliability of the reported result and reducing the impact of artifacts introduced by WGA, PGA and/or NGS. Methods relying on array CGH generally do not provide base frequency information. Methods relying on SNP arrays generally do not provide base frequencies for the 4 bases (but only for 2 bases, cf. B-allele frequencies).

Hence, every segment is characterized by a read count (corrected for the positional information) and the observed 4-base frequencies.

In a next step, nearby segments (consecutive or closely adjacent according to their position in the chromosome) are grouped into 1 master segment according to the presence of a similar pattern. As an example, 100 consecutive segments are grouped into 1 master segment, as every segment contains a similar read count and the base frequencies observed in each of the 100 segments cluster together in a specific band pattern. If this band pattern for the base frequencies is e.g. 0, 33%, 66% and 100% and the average read count across the 100 segments is about 50% higher as compared to the rest of the genome, this indicates that the identified master segment displays a CNV (i.e. a triploid master segment). The fact that both read count and 4-base frequencies are combined in the interpretation increases the likelihood that the reported result is correct. The fact that the data from multiple consecutive segments are combined minimizes the influence of an artifact in an individual segment introduced by WGA, PGA or NGS on the reported result. As array CGH does not provide base frequency information, the diagnostic result will be less reliable, as it is not the result from 2 different sources of information. As SNP arrays do not provide 4-base frequency, the reported result will be less reliable, as there was less information available.

The same methodology can be expanded towards:
- screening for chromosomal CNVs
- diagnosis of deletions or amplifications
- diagnosis of balanced translocations or inversions
- diagnosis of unbalanced translocations
- different fields, cf. non-invasive prenatal testing, cancer, epigenomic profiling using methylation-sensitive enzymes, ...

### Example 4: Diagnosis of a risk allele for a dominant monogenic disorder in a preimplantation embryo in less than 24h.

In general, monosomy for any of the autosomes is not viable and transfer of such an embryo is unlikely to result in a pregnancy. Uniparental disomy for some autosomes can be viable, and transfer of such an embryo may result in a pregnancy. However, the foetus or child is more likely to be abnormal and hence it would not be recommended to transfer such an embryo. A high degree of consanguinity is likely to be detected as uniparental disomy for a significant portion of the genome

In certain cases, it is important to test the DNA of a preimplantation embryo for the presence of risk alleles in less than 24 hours, to enable transfer an embryo that does not contain a certain risk allele within the same cycle.
In the present case, one of the 2 parents (parent 1) carries one risk allele of a dominant monogenic disorder and is affected. The other parent (parent 2) carries 0 risk alleles of the dominant monogenic disorder and is healthy. One of the 2 parents from parent 1 (grandparent 1) carries two risk alleles of the dominant monogenic disorder and is affected. The other parent from parent 1 (grandparent 2) carries 0 risk alleles of the dominant monogenic disorder and is healthy. In this case it is important to determine in the preimplantation embryo if the risk allele from parent 1 (which was inherited from grandparent 1) is inherited in the embryo or not.

For each segment of the reduced representation library, the NGS data are integrated into a summarising dataset. As described in example 2, for every segment, the number of reads is counted. As described in example 2, for every position in the segment, the frequency of each of the 4 bases is calculated, and for every segment, the observed base frequencies for the 4 bases are counted.

In addition, for every variant in the embryo with a base frequency above a lower noise level (e.g.>10%) and optionally below an upper noise level (e.g. <90%), the probability that the variant has a paternal or a maternal origin (i.e. the parental probabilities), and a grandpaternal or grandmaternal origin (i.e. the grandpaternal probabilities) can be determined. However, a definitive call on the ancestral origin is not made, because the reads of that variant position in the embryo may be perturbed by artifacts related to WGA, PGA or NGS. Likewise the reads of that variant position in the parents and grandparents may be perturbed by artifacts related to PGA or NGS. Instead, the ancestral probabilities are calculated and a definitive call will be made based on the assembly of consecutive segments into a master segment with an overall similar profile in terms of number of reads, 4 base frequency and ancestral probability. It is possible that at one position, all 4 bases have a frequency above the lower noise level and hence 4 possible variants are identified. In that case, it is realistic to assume that at least 1 of the variants is introduced by an artifact related to WGA, PGA and/or NGS. Traditional methods would only consider the 1 or 2 variants with the highest base frequency. However, there is no guarantee that the highest frequency variants are not introduced by an artifact. Therefore, a definitive call will be made based on the assembly of consecutive segments into master segments with an overall similar profile in terms of number of reads, 4 base frequency and ancestral probability. This is different from methods relying on SNP arrays, in which only the A or B allele frequency is calculated (as only 2 bases can be detected). Moreover, it also differs from methods relying on discrete SNP calls, in which the base frequencies are artificially set to 0, 50 or 100%, thereby removing valuable information that can no longer be used for the subsequent pattern recognition. Note that a variant can also be a deletion or an insertion of 1 or more consecutive bases, and that to enable its use in our method, this deletion or insertion should not have a specific population frequency that is sufficiently high to have been included in the SNP array.

Hence, every segment is characterized by a read count (optionally corrected for the positional information) and the observed base frequencies. Furthermore, every variant is characterized by ancestral probabilities.

In a next step, nearby segments (according to the reference genome) are grouped into 1 master segment according to the presence of a similar pattern. As an example, 100 consecutive segments are grouped into 1 master segment, as every segment contains a similar read count, the 4 base frequencies observed in each of the 100 segments cluster together in a specific band pattern and the overall grandparental 1 probability is high across the variants in the master segment. The fact that read count, 4 base frequencies and ancestral probabilities are combined in the interpretation increases the likelihood that the reported result is correct. The fact that the data from multiple consecutive segments are combined minimizes the influence of an artifact in an individual segment introduced by WGA, PGA or NGS on the reported result. As SNP arrays do not provide base frequency information for the 4 bases, the diagnostic result will be less reliable, as there was less information available. As traditional haplotyping methods rely on discrete SNP calls and a discrete parental origin prior to segment assembly, the diagnostic result based on such a method will be less reliable, as there was less information available for the pattern recognition and the discrete SNP calls may be perturbed by artifacts related to WGA, PGA and/or NGS.
Note that the chance for artifacts in parental and grandparental samples is smaller, because neither the parental nor the grandparental samples require WGA, and hence there are no WGA-induced artifacts.

Using this method, it can be determined if there is a master segment present in the embryo that has a most likely grandparental 1 origin, and that covers the genomic location of the risk allele. If that is the case, it would be not recommended to select that embryo for transfer.

The same methodology can be expanded towards:
- diagnosis of autosomal dominant or recessive disorders
- diagnosis of X or Y-linked, dominant or recessive disorders
- diagnosis of disorders when other pedigree members are available, e.g. parental siblings, siblings, ...
- diagnosis of chromosomal recombination sites using different siblings and/or embryos and/or gametes

### Example 5: Identification of the origin of the chromosomal aberration.

In certain cases, it is important to identify the most likely parental origin of the segment(s) in the pericentromeric region (the region of the chromosome that contains the centromere), as well as the most likely ploidy state of the pericentromeric region for each of the chromosomes. Information on the parental origin and the ploidy state of the pericentromeric region allows to identify the origin of a chromosomal aberration. This may be relevant to deduce whether there is a risk that the chromosomal aberration will be found throughout the embryo.

### 1. Non-disjunction error in meiosis I

This is exemplified by an embryo for which there were 3 master segments identified in the pericentromeric region of a certain chromosome:
- a first master segment is most likely to be paternal and most likely to have a ploidy state of 1
- a second master segment is most likely to be maternal and most likely to have a ploidy state of 1
- a third master segment is most likely to be maternal and most likely to have a ploidy state of 1
Note that this reflects a scenario in which the second and third master segment are most likely to be derived from the 2 different copies of that chromosome in the mother. The presence of the 2 different maternal master segments in the pericentromeric region indicates that the aberration is most likely to originate from a non-disjunction error in meiosis I in the oocyte. Hence, the aberration is most likely to be present throughout the embryo, and it would be not advisable to select the embryo for embryo transfer.

This is opposed to aberrations that would have originated from a postzygotic error in the segregation of the chromosomes (i.e. during mitosis), in which case the embryo biopsy material would not have been representative for the other cells of the embryo.

### 2. Error in meiosis II

Another example is given by an embryo for which there were 2 master segments identified in the pericentromeric region of a certain chromosome, and 3 master segments identified in a distal region of the same chromosome:
For the segments in the pericentromeric region:
- a first master segment is most likely to be paternal and most likely to have a ploidy state of 1
- a second master segment is most likely to be maternal and most likely to have a ploidy state of 2
Note that this reflects a scenario in which the second, diploid master segment in the pericentromeric region is most likely to be derived from a single copy of that chromosome in the mother.
For the master segments in the distal region:
- a first master segment is most likely to be paternal and most likely to have a ploidy state of 1
- a second master segment is most likely to be maternal and most likely to have a ploidy state of 1
- a third master segment is most likely to be maternal and most likely to have a ploidy state of 1
Note that this reflects a scenario in which the second and third segment in the distal region are most likely to be derived from the 2 different copies of that chromosome in the mother.
The presence of only 1 maternal master segment with a ploidy state of 2 in the pericentromeric region, along with 2 different maternal master segments with a ploidy state of 1 in a distal region indicates that the aberration is likely to originate from an error in meiosis II in the oocyte. Hence, the aberration is most likely to be present throughout the embryo, and it would be not advisable to select the embryo for embryo transfer.

This is opposed to aberrations that would have originated from a postzygotic error in the segregation of the chromosomes (i.e. during mitosis), in which case the embryo biopsy material would not have been representative for the other cells of the embryo.

The outcome of the analyses is provided in terms of "most likely to have a ploidy state of x" and "most likely to have a paternal origin"

Apart from identifying the origin of the chromosomal aberration (see previous examples), information on the ancestral origin of the pericentromeric region can also be applied to identify balanced structural chromosome abnormalities.

### Example 6: Identification of balanced structural chromosome abnormalities

In certain cases, it is important to identify balanced structural chromosome abnormalities, such as balanced translocations or inversions, because such abnormalities can cause repeated miscarriage or repeated miscarriage.
In the present case a parent (e.g. father) that carries a balanced chromosomal inversion in one of the two copies of a certain chromosome, which was inherited from a grandparent (e.g. grandfather).
By applying the method on the father and the 2 paternal grandparents, it can be identified which pericentromeric master segment in the father is most likely to be inherited from the grandfather. Hence, it can be deduced which pericentromeric master segment is most likely to be present on the paternal chromosome carrying the inversion.
By comparing with the most likely paternal pericentromeric master segment of that chromosome in the embryo, it can be deduced whether the embryo is most likely to have inherited the chromosome with the inversion and whether it is advisable to reject the embryo for embryo transfer.
Similarly, the method can be applied to identify the presence of balanced chromosomal translocations.
Unbalanced structural chromosome abnormalities can be identified based on the presence of (sub)chromosomal CNVs, as exemplified before.

### Example 7: Epigenomic profiling of Circulating Tumour Cells (CTCs)

In certain cases, it is important to screen for epigenetic alterations, since epigenetic alteration (in particular hypermethylation and hypomethylation) may play an important role in the transformation of a cell and cancer. Knowledge on the epigenetic profile (and evolution thereof) of cancer can be developed as a tool to e.g. diagnose the presence of a cancer, determine the stage of a particular cancer, make a therapy decision, evaluate the effectiveness of a specific therapy, and make a molecular prognosis of the survival time of the patient.

Methylation-sensitive and methylation-dependent restriction enzymes can be used to create a reduced representation library on a CTC that was isolated at a specific timepoint. Depending on the methylation of the RERS, some fragments will not be present in the reduced representation library. Upon applying NGS, clustering of the segments into master segments can be performed, and an epigenetic profile can be established, in which the epigenetic profile is described by e.g. number of reads assigned to each master segment.
It can also be determined e.g. which of the expected segments were not detected in the sequence read data and hence could not be clustered into the master segment. This can be determined for each of the segments individually, or on a genome-wide scale. The latter can be described as a total number of missing segments.

The absence of these segments can be the effect of an artifact or be e.g. caused by the methylation of the RERS of the methylation-sensitive RE. It can be expected that the number of artifacts will be similar across different CTCs, and hence that changes in the total number of missing segments represent changes in the overall methylation profile of the CTC as compared to a reference. Hence, this reflects another metric describing the epigenetic profile of the CTC.

The same method can be applied to perform epigenetic profiling of:
- isolated CTCs,
- exosomes,
- circulating tumor DNA in body fluids, such as urine, blood, saliva, cerebrospinal fluid
- circulating foetal cells or free foetal DNA in blood
- biopsy material from a preimplantation embryo
- biopsy material from a foetus, new born, or individu (cf. children, parents, grandparents, ...), or horse, cow, pig, ...
- tumour cells present in a biopsy tissue sample, or isolated from a tissue slice (Fresh Frozen Tissue or Formalin-Fixed Paraffin-Embedded Tissue)
- ...

### Example 8: genomic CNV profile of a CTC

The method described for determination of (sub)chromosomal CNVs in an embryo biopsy can also be applied to determine the genomic CNV profile of a CTC. Knowledge on the genomic CNV profile (and evolution thereof) of cancer cells can be developed as a tool to e.g. diagnose the presence of a cancer, determine the stage of a particular cancer, make a therapy decision, evaluate the effectiveness of a specific therapy, and make a molecular prognosis of the survival time of the patient.

### Example 9: Mosaicism

In some cases it may be beneficial to evaluate if the analysis on a single blastomere cell is representative for the other cells of the embryo. In such cases it is relevant to identify if the aberration is most likely to originate from an error in meiosis I or meiosis II. If the aberration is most likely to have such a meiotic origin, then there is high chance there is no mosaicism in the embryo for that particular aberration. In that case it is most likely that the aberration is present throughout the embryo. Inversely, if the aberration is most likely to have a mitotic origin, there is a high chance for mosaicism in the embryo for that particular aberration.

In some cases it may be required to analyse subchromosomal CNV mosaicism in trophectoderm biopsy containing a few cells (e.g. 5 cells). The example is given in which one of the cells contains a subchromosomal trisomy due to a mitotic event (i.e. the event has no meiotic origin, and hence is not present in all the cells), and assumes that the subchromosomal trisomy is composed of 2 paternal copies and 1 maternal copy.
When applying the described method to such a sample, it will result in the identification of a master segment (or a set of master segments) covering that subchromosomal region, in which the master segment with a most likely paternal origin has a ploidy state of about 1.2 (i.e. 6 paternal copies in 5 cells). Based on reference data, it can be deduced if the ploidy state of 1.2 is significantly different from 1. In that case, the probability can e.g. be identified that at least one of the cells has a paternal ploidy state of at least 2 for that segment.

The same method can be applied to:
- identification of both chromosomal as well as subchromosomal mosaic CNVs
- identification of mosaic CNVs in any mixture of cells (e.g. trophectoderm biopsy, CTCs, cancer cells, tumor tissue cells, mixtures of healthy and affected cells, ...) containing at least 2 cells.

Other cases may require the identification of CNVs in foetal cells or cell-free foetal DNA present in maternal blood. If the foetal DNA fraction is sufficiently high, CNVs in the foetal DNA will be identified as master segments with a ploidy state that is significantly different from 2. Note that this application does not require information on the paternal DNA.

When paternal DNA is available, the described method can be applied to blood of a pregnant woman and blood of the father of the foetus. This will enable the identification of master segments that have a most likely paternal origin. The cell-free fetal DNA is only a fraction of the total DNA in the sample (in which the majority is maternal DNA), and hence the master segments with most likely paternal origin will have an overall low read count as compared to the master segments with most likely maternal origin. Across the most likely paternal master segments, it can be evaluated if any of the most likely paternal segments display a chromosomal or subchromosomal CNV. Note that a comparison of read count associated with most likely paternal segments vs. most likely maternal segments indicates the foetal DNA fraction in the maternal blood.

The same method can be applied to:
- identification of foetal CNV mosaicism in a mixture of circulating foetal cells or cell-free foetal DNA and maternal DNA in which there is a twin pregnancy
- identification of the presence of risk alleles related to inheritable disorders in the foetus or foetuses
- identification of the presence of inversions, balanced translocations, unbalanced translocations, subchromosomal CNVs, chromosomal CNVs.

Other cases may require the identification of CNV mosaicism in CTCs or cell-free circulating tumor DNA present in blood. If the tumour DNA fraction is sufficiently high, CNVs in the tumour DNA will be identified as master segments with a ploidy state that is significantly different from 2.

The same method can be applied to:
- analysis of exosomes present in blood, and exosomes isolated from blood.
- analysis of CTCs or cell-free tumour DNA in other body fluids (saliva, cerebrospinal fluid, urine, serum)

### Example 10: HLA matching

The method as explained in the previous examples can also be applied to human leucocyte antigen (HLA) matching, with the aim of isolating cord blood stem cells at birth for transplantation to an existing child with a serious blood related illness. Traditional methods require the development of a patient-specific test that covers a sufficient number of linked markers in the HLA region. The described method is generic and does not require the development of patient-specific tests. Moreover, due to the genome-wide distribution of the fragments, the number of linked markers is much higher than the 4-10 markers that are typically used in the traditional methods.

### Example 11: Noise typing to support analysis of the target genome.

This is exemplified in a scenario in which a certain master segment was identified, the overall parental probability of the master segment was determined, and it was found that the master segment was most likely to be paternal. For the corresponding genomic region, no most likely maternal segment was identified, suggesting that there was only a paternal contribution for that genomic region.
For each of the composing segments, it can be analyzed if the parental probability of the segment was in agreement with the overall paternal probability of the master segment. If one would hypothesize that there should have been a maternal contribution to that genomic region, this would contrast with the observed systematic, high frequency ADO for such a maternal contribution across that genomic region. This would indicate that the hypothesis is not correct, and that there was no maternal contribution for that master segment. This exemplifies how ADO rates can be used to confirm the absence of a parental (maternal) segment.
If the master segment would have a ploidy state of about 1 and no 4-base frequencies that cluster in the 25%, 33, 50%, 66% nor 75% region, this may indicate a unipaternal monosomy, while a unipaternal disomy can be expected if the segment has a ploidy state of 2 and no 4-base frequencies that cluster in the 25%, 33, 50%, 66% nor 75% region. Hence, the typing of noise can further support the analysis of the target genome.

The same method can be applied to:
- master segments with a most likely maternal origin
- support other analyses of the target genome

### Example 12: Noise typing to identify a sample switch

This is exemplified in a scenario in which a set of master segments was identified, and the overall parental probability of each of the master segments was determined. It is expected that there is a random occurrence of ADI, and hence a random, low frequency discordance in parental probability across the composing segments and their corresponding master segment. Likewise, it would be expected that there is a high parental probability for each of the master segments. However, if there has been a sample switch (e.g. the wrong father, or an embryo from a different family), this will lead to the systematic occurrence of ADI, and hence a systematic, high frequency discordance in parental probability across the composing segments and their corresponding master segment. Likewise, this would lead to a low parental probability for each of the master segments. Hence, the typing of noise can identify the presence of a sample switch.

### Example 13: Construction of reduced representation library

Reference genome GRCh38 build 38 is taken. When digested with EcoRI and PstI, this generates about 2,169K DNA fragments, of which about 897K fragments are dual-ended (i.e. contain EcoRI on one side and PstI on the other side). After adapter ligation and suppression PCR, the adapter-ligated dual-ended fragments will have been exponentially enriched in the pool of DNA fragments. When applying an additional size selection step selecting for DNA fragments in the range of 250 to 450 bp (given sizes exclude the adapters), the pool is further reduced to about 100K fragments and spans about 34.7Mb of the genome. As such, the original 3Gb genome has been reduced by about 89-fold.

In another example, again the reference genome GRCh38 build 38 is taken. When digested with EcoRI and XhoI, this generates about 969K DNA fragments, of which about 192K fragments are dual-ended (i.e. contain EcoRI on one side and XhoI on the other side). After adapter ligation and suppression PCR, the adapter-ligated dual-ended fragments will have been exponentially enriched in the pool of DNA fragments. When applying an additional size selection step selecting for DNA fragments in the range of 250 to 450 bp (given sizes exclude the adapters), the pool is further reduced to about 10K fragments and spans about 3.6Mb of the genome. As such, the original 3Gb genome has been reduced by about 860-fold.

### Example 14: Preimplantation genetic testing

In a first step, the samples are prepared for sequencing and sequenced, as schematically depicted in Figure 1.
1. The samples may consist of embryo biopsies (e.g. 1 blastomere isolated from a cleavage-stage embryo, or e.g. 2-10 trophectoderm cells isolated from a blastocyst-stage embryo) and genomic DNA isolated from family members, e.g. the female patient undergoing an In Vitro Fertilization treatment, the male patient from whom sperm is used for fertilization of the oocyte from the female patient, or phasing reference(s) (which can be e.g. an affected child from the female and male patient, or e.g. the parents of the patient that carries a certain risk allele). Each embryo biopsy is whole genome amplified using MDA (or PCR-based amplification methods such as PicoPlex, SurePlex, MALBAC), and the whole genome amplified material is digested using 2 restriction enzymes. The genomic DNA isolated from the family members is also digested using (preferably the same) 2 restriction enzymes.
2. After this double digestion, 2 adapters (1 adapter for each restriction enzyme) are added, and the adapters are ligated to the DNA fragments using a DNA ligase. At this point, the mixture is composed of dual-ended and same-ended adapter-ligated fragments.
3. During a subsequent PCR step, the same-ended adapter-ligated fragments will preferentially form intramolecular hairpin loops, and will therefore not be efficiently amplified, in contrast to the dual-ended adapter-ligated fragments. After a number of PCR cycles (typically between 5 and 50), the dual-ended adapter-ligated fragments will have been significantly enriched over the same-ended fragments. In addition, at least 1 of the primers carries a sample-specific barcode and will have introduced this barcode into the dual-ended adapter-ligated fragments. Using this barcode, it will be possible to uniquely identify each sample in the pool of samples that will be sequenced in a single NGS run. Alternatively, the sample-specific barcodes may already have been present in 1 or both adapters and hence do not need to be introduced via the PCR primers.
4. After PCR cycling, the PCR product can be purified and optionally this is accompanied by a size-selection to preferentially purify PCR products of a certain length.
5. Finally, the purified PCR products are pooled and the sequencing is performed according to the manufacturer's instructions.

In a second step, the output data of the NGS platform are processed, as depicted in Figure 2.
1. The output data of the NGS platform is converted and demultiplexed into per-sample FASTQ files containing every read that is assigned to a certain sample (according to the sample-specific barcode). The assigned reads are subsequently mapped onto a reference genome. This results in a set of segments to which reads are assigned, and these mapping data are stored in one or more BAM files. Alternatively, the output data of the NGS platform can be directly converted, demultiplexed and mapped into BAM files (i.e. without the intermediate step of making a FASTQ file), which may offer benefits in terms of the total time needed to perform the processing.
2. For each segment, the sequencing data of the associated reads are integrated into a summarizing dataset containing metrics. These raw sequencing metrics may be
   a. positional information of the segment,
   b. observed frequencies of one, two or three particular base(s) in the fragment or at one or more particular position(s) in the fragment (which is also termed base frequency),
   c. observed frequencies of the four bases in the fragment or at one or more particular position(s) in the fragment (which is also termed the 4-base frequency),
   d. the number of reads mapped to that segment (which is also termed read count),
   e. the normalized number of reads (which is also termed normalized read count), in which the normalization may be based on total number of reads mapped to a certain sample and/or the GC content of the segment and/or the GC content of the DNA sequence surrounding the segment in the reference genome and/or observed read counts for that particular segment in a historical dataset and/or any other normalization method
   f. ancestral origin of the segment or a particular position in the segment, in which ancestral origin can be deduced using discrete genotyping algorithms and textbook knowledge (e.g. if standard genotyping algorithms indicate that the father is homozygous AA for a certain position, the mother is heterozygous AC for the same position, and the embryo biopsy is heterozygous AC for the same position, it can be deduced that the reads in the embryo containing a C originate from DNA that was inherited from the mother, and hence that that particular position has a maternal origin).
   g. ancestral probability of the segment or a particular position in the segment, in which ancestral probability is deduced from base frequencies or 4-base frequencies instead of discrete genotyping algorithms, e.g. if the father is about 90-100% A for a certain position, the mother is about 45-55% A and 45-55% C for the same position, and the embryo biopsy is about 45-55% A and 45-55% C for the same position, it can be deduced that the reads in the embryo containing a C most likely originate from DNA that was inherited rom the mother. However, if due to noise in the single cell sequencing data the embryo biopsy is about 80-90% A and only about 10-20% C for the same position, the reads in the embryo containing a C may have originated from DNA that was inherited from the mother, but may also be caused by artifacts related to the preceding Whole Genome Amplification step. As such, the maternal probability of the segment will be lower in the second case as compared to the first case.
   h. quality scores for mapping and/or base-calling,
   i. and/or any metric derived thereof.
3. These metrics are used in a segmentation model that clusters non-overlapping, nearby segments with similar raw metrics into master segments.
   a. Only segments that are consecutive or in relatively close proximity and on the same chromosome in the reference genome can be assembled into 1 master segment. As such, clustering is typically performed per chromosome.
   b. Consecutive segments that have similar raw sequencing metrics are likely to be assembled into 1 master segment. For instance, segment A having 99 reads, base frequencies that cluster close to 0, 50 and 100%, and a high paternal probability are likely to be assembled with segment B having 100 reads and base frequencies that cluster close to 0, 50 and 100%, and also a high paternal probability.
   c. Note that this does not exclude the chance that consecutive fragments may have contradictory raw sequencing metrics (e.g. fragment C having a very high paternal probability, and fragment D having a low paternal probability) and are still clustered into 1 master segment, provided that their clustering is supported by a sufficient number of surrounding segments that have similar raw sequencing metrics and were therefore also assigned to the same master segment. Contradictory raw sequencing metrics may be caused by artifacts during WGA, PGA or NGS, but the fact that multiple fragments are assembled into a master segment filters out the impact of such artifacts on the final, discrete call for the master segment.
   d. The clustering can be driven by a single metric (e.g. read count, or base frequencies, or 4-base frequencies, or ancestral origin, or ancestral probability or any other metric) or a combination of multiple metrics (e.g. read count and base frequencies and/or 4-base frequencies, ancestral origin and ancestral probability or any other combination of 2 or more metrics)
   e. The master segments are characterized by metrics derived from the raw metrics. For continuous metrics (like e.g. read count), this can be e.g. the average or median raw metric across the assigned segments, while for discrete metrics (like e.g. ancestral origin), this can be the most frequently observed value across the assigned segments. Alternative methods to calculate the overall metric for a master segment exist.
   f. The segmentation model aims to identify master segments that are biologically relevant. It is e.g. most likely that the number of recombination sites (which can be identified as e.g. a position where a master segment originating from the father of the male patient is adjacent to a master segment originating from the mother of the male patient) is low (typically between 0 and 10 per chromosome) and correlated with the size of the chromosome. It is also e.g. unlikely that a single chromosome would be composed of many master segments from which the overall normalized read count is alternating across the master segments (e.g. master segment 1 has an overall normalized read count indicative of disomy, an adjacent master segment 2 has an overall normalized read count indicative of trisomy, an adjacent master segment 3 has an overall normalized read count indicative of disomy, an adjacent master segment 4 has an overall normalized read count indicative of trisomy and an adjacent master segment 5 has an overall normalized read count indicative of disomy). Alternative criteria to include biological relevance in the segmentation model exist.
4. A final, discrete DNA call can be made based on the identified master segments and their summarizing metrics. The final discrete DNA call may involve probability-based identification of chromosomal recombination sites, (sub)chromosomal copy number variations, deletions, unbalanced or balanced translocations, inversions, amplifications, the presence of risk alleles for inherited disorders, errors in meiosis I or meiosis II, balanced structural chromosome abnormalities; epigenomic profiles of cells, mosaicisms, human leucocyte antigen (HLA) matches and/or noise typing.

## Claims

1. A method of target DNA genome analysis, which method comprises the steps of:
- obtaining raw metrics for non-overlapping segments using a sequencing process applied on a reduced representation library of said target DNA genome,
wherein said reduced representation library has been enriched for target DNA genome fragments having two boundaries defined by predetermined DNA sequences;
- using a reference genome, clustering non-overlapping segments, which are consecutive or closely adjacent according to their position in the chromosome in the reference genome, with similar raw metrics to assemble said segments into master segments;
- providing metrics describing the master segments in which said metrics include inferred boundaries of one or more master segments, number of observed reads in one or more master segments, observed 4-base frequencies in said one or more master segments, or ancestral probability for one or more of said master segments; and
- making a final discrete DNA call based on the clustering of segments;
wherein said raw metrics include base frequency, read count, and ancestral information.

2. The method according to claim 1, wherein said reduced representation library has been enriched for target DNA genome fragments with boundaries defined by two different predetermined DNA sequences.

3. The method according to any one of the previous claims,
wherein said predetermined DNA sequences comprise a restriction enzyme recognition site.

4. The method of claim 3, wherein enrichment of target DNA genome fragments has been performed using a restriction enzyme.

5. The method according to any one of the previous claims,
wherein the target DNA genome is derived from one to ten cells or one to 1000 cells.

6. The method according to claim 5, wherein the target DNA genome is derived from cells from a trophectoderm biopsy, one or two polar bodies, foetal cells or cell-free foetal DNA found in the maternal peripheral blood circulation, or circulating tumour cells or cell-free tumour DNA.

7. The method according to any one of the previous claims,
wherein the method involves preimplantation genetic screening, preimplantation genetic diagnosis, cancer screening, cancer diagnosis, cell typing or ancestral origin identification.

8. The method according to claim 6, wherein the target DNA genome is derived from one or two blastomeres, and wherein the method involves preimplantation genetic screening or preimplantation genetic diagnosis.

9. The method according to any one of the previous claims,
wherein the reduced representation library has been generated using a wholly or partially amplified target DNA genome.

10. The method according to claim 1, wherein the final discrete DNA call involves probability-based identification of:
chromosomal recombination sites, (sub)chromosomal copy number variations, deletions, unbalanced or balanced translocations, inversions, amplifications, the presence of risk alleles for inherited disorders, errors in meiosis I or meiosis II, balanced structural chromosome abnormalities; epigenomic profiles of cells, mosaicisms, human leucocyte antigen (HLA) matches, or noise typing.

11. The method according to claim 1, wherein the final discrete DNA call involves determining copy number and ancestral origin of the master segments.

12. The method according to any one of the previous claims wherein the clustering uses an in silico simulated reference genome.

13. The method according to any one of the previous claims wherein the clustering into master segments uses pedigree information.

14. The method according to any one of the previous claims wherein the clustering into master segments is ancestral probability-based and derived from pedigree information.

15. The method according to any one of the previous claims, wherein the target DNA genome is a foetal DNA genome and wherein said foetal DNA genome is derived from a fluid sample obtained from a female pregnant with a foetus having said foetal DNA genome.

16. The method according to claim 15, further comprising a size selection step prior to performing the sequencing process, wherein said size selection step enriches fragments having a size of less than 250 basepairs.

## Patentansprüche

1. Ein Verfahren zur Genomanalyse einer Ziel-DNA, wobei das Verfahren folgende Schritte umfasst:
- Erhalten roher Messgrößen für nicht-überlappende Segmente unter Verwendung eines Sequenzierungsprozesses, angewendet an einer Reduced-Representation-Library des Genoms der Ziel-DNA,
- wobei die Reduced-Representation-Library für Genomfragmente einer Ziel-DNA mit zwei Grenzlinien, definiert durch vorbestimmte DNA-Sequenzen, angereichert wurde;
- Verwenden eines Referenzgenoms, Clustern von nicht-überlappenden Segmenten, die aufeinander folgend oder dicht aneinander liegend sind gemäß ihrer Position im Chromosom im Referenzgenom, mit ähnlichen rohen Messgrößen, um die Segmente in Master-Segmente zusammenzufügen;
- Bereitstellen von Messgrößen, die die Master-Segmente beschreiben, in denen die Messgrößen abgeleitete Grenzlinien von einem oder mehreren Master-Segmenten, eine Anzahl von wahrgenommenen Ablesungen in einem oder mehreren Master-Segmenten, wahrgenommene 4-Basen-Frequenzen im einen oder den mehreren Master-Segmenten, oder durch Vorfahren bedingte Wahrscheinlichkeit für einen oder mehreren der Master-Segmente einschließen; und
- Anstellen eines finalen diskreten DNA-Abrufs basierend auf dem Clustern von Segmenten;
wobei die rohen Messgrößen Basenfrequenz, Anzahl der Ablesungen und von Vorfahren abgeleitete Informationen einschließen.

2. Das Verfahren nach Anspruch 1, wobei die Reduced-Representation-Library für Genomfragmente einer Ziel-DNA mit Grenzlinien, definiert durch zwei verschiedene vorbestimmte DNA-Sequenzen, angereichert wurde.

3. Das Verfahren nach irgendeinem der vorigen Ansprüche, wobei die vorbestimmten DNA-Sequenzen eine Erkennungsstelle durch Restriktionsenzymen umfassen.

4. Das Verfahren nach Anspruch 3, wobei die Anreicherung von Genomfragmenten einer Ziel-DNA unter Verwendung eines Restriktionsenzyms durchgeführt wurde.

5. Das Verfahren nach irgendeinem der vorigen Ansprüche, wobei das Genom der Ziel-DNA von einer bis zehn Zellen oder einer bis 1.000 Zellen herrührt.

6. Das Verfahren nach Anspruch 5, wobei das Genom der Ziel-DNA von Zellen von einer Trophektodermbiopsie, einem oder zwei Polkörpern, fetalen Zellen oder zellfreier fetaler DNA , gefunden im mütterlichen peripheren Blutkreislauf, oder zirkulierenden Tumorzellen oder zellfreier Tumor-DNA herrührt.

7. Das Verfahren nach irgendeinem der vorigen Ansprüche, wobei das Verfahren präimplantive genetische Erkennung, präimplantive genetische Diagnostik, Erkennung von Krebs, Krebsdiagnose, Zelltypisierung oder von Vorfahren abgeleitete Abstammungsbestimmung umfasst.

8. Das Verfahren nach Anspruch 6, wobei das Ziel-DNA-Genom von einem oder zwei Blastomeren herrührt, und wobei das Verfahren präimplantive genetische Erkennung oder präimplantive genetische Diagnostik involviert.

9. Das Verfahren nach irgendeinem der vorigen Ansprüche, wobei die Reduced-Representation-Library unter Verwendung eines zur Gänze oder teilweise amplifizierten Genoms einer Ziel-DNA generiert wurde.

10. Das Verfahren nach Anspruch 1, wobei der finale diskrete DNA-Abruf wahrscheinlichkeitsbasierte Identifikation umfasst, von: chromosomalen Rekombinationsstellen, (sub)chromosomalen Kopienzahlvariationen, Deletionen, unbalancierten oder balancierten Translokationen, Inversionen, Amplifikationen, Anwesenheit von Risikoallelen für vererbte Störungen, Fehlern in Meiose I oder Meiose II, balancierten strukturellen Chromosomenabweichungen; epigenomischen Zellprofilen, Mosaizismen, Humane-Leukozyten-Antigen(HLA)-Übereinstimmungen, oder Rausch-Typisierung.

11. Das Verfahren nach Anspruch 1, wobei der finale diskrete DNA-Abruf das Bestimmen von Kopienzahl und von Vorfahren abgeleitete Abstammung der Master-Segmente umfasst.

12. Das Verfahren nach irgendeinem der vorigen Ansprüche, wobei das Clustern ein *in silico* simuliertes Referenzgenom verwendet.

13. Das Verfahren nach irgendeinem der vorigen Ansprüche, wobei das Clustern in Master-Segmente Abstammungsinformationen verwendet.

14. Das Verfahren nach irgendeinem der vorigen Ansprüche, wobei das Clustern in Master-Segmente auf durch Vorfahren bedingte Wahrscheinlichkeit basiert und von Abstammungsinformationen herrührt.

15. Das Verfahren nach irgendeinem der vorigen Ansprüche, wobei das Genom der Ziel-DNA ein Genom einer fetalen DNA ist und wobei das Genom der fetalen DNA von einer Flüssigkeitsprobe herrührt, erhalten von einer Frau, die mit einem Fetus schwanger ist, der das Genom der fetalen DNA hat.

16. Das Verfahren nach Anspruch 15, welches ferner einen Schritt der Größenauswahl vor der Durchführung des Sequenzierungsprozesses umfasst, wobei der Schritt der Größenauswahl Fragmente anreichert, die eine Größe von weniger als 250 Basenpaaren haben.

## Revendications

1. Procédé d'analyse du génome d'ADN cible, lequel procédé comprend les étapes consistant à :
- obtenir des métriques brutes pour des segments qui ne se chevauchent pas à l'aide d'un procédé de séquençage appliqué à une banque de représentation réduite dudit génome d'ADN cible,
dans lequel ladite banque de représentation réduite a été enrichie pour des fragments de génome d'ADN cible ayant deux limites définies par des séquences d'ADN prédéterminées ;
- en utilisant un génome de référence, regrouper les segments qui ne se chevauchent pas, qui sont consécutifs ou étroitement adjacents en fonction de leur position dans le chromosome dans le génome de référence, avec des métriques brutes similaires pour assembler lesdits segments en segments maîtres ;
- fournir des métriques décrivant les segments maîtres dans lesquels lesdites métriques comprennent les limites inférées d'un ou plusieurs segments maîtres, le nombre de lectures observées dans un ou plusieurs segments maîtres, les fréquences des 4 bases observées dans lesdits un ou plusieurs segments maîtres, ou la probabilité ancestrale pour un ou plusieurs desdits segments maîtres ; et
- faire un appel d'ADN discret final basé sur le regroupement de segments ;
dans lequel lesdites métriques brutes comprennent la fréquence des bases, le nombre de lectures et les informations ancestrales.

2. Procédé selon la revendication 1,
dans lequel ladite banque de représentation réduite a été enrichie pour des fragments de génome d'ADN cible avec des limites définies par deux séquences d'ADN prédéterminées différentes.

3. Procédé selon l'une quelconque des revendications précédentes,
dans lequel lesdites séquences d'ADN prédéterminées comprennent un site de reconnaissance par des enzymes de restriction.

4. Procédé selon la revendication 3, dans lequel l'enrichissement de fragments de génome d'ADN cible a été effectué en utilisant une enzyme de restriction.

5. Procédé selon l'une quelconque des revendications précédentes,
dans lequel le génome d'ADN cible est dérivé d'une à dix cellules ou d'une à 1000 cellules.

6. Procédé selon la revendication 5, dans lequel le génome d'ADN cible est dérivé de cellules provenant d'une biopsie du trophectoderme, d'un ou deux corps polaires, de cellules fœtales ou d'ADN fœtal exempt de cellules trouvées dans la circulation sanguine périphérique maternelle ou de cellules tumorales en circulation ou d'ADN tumoral exempt de cellules.

7. Procédé selon l'une quelconque des revendications précédentes,
dans lequel le procédé implique le dépistage génétique préimplantatoire, le diagnostic génétique préimplantatoire, le dépistage du cancer, le diagnostic du cancer, le typage cellulaire ou l'identification de l'origine ancestrale.

8. Procédé selon la revendication 6, dans lequel le génome d'ADN cible est dérivé d'un ou deux blastomères, et dans lequel le procédé implique un dépistage génétique préimplantatoire ou un diagnostic génétique préimplantatoire.

9. Procédé selon l'une quelconque des revendications précédentes,
dans lequel la banque de représentation réduite a été générée en utilisant un génome d'ADN cible totalement ou partiellement amplifié.

10. Procédé selon la revendication 1, dans lequel l'appel d'ADN discret final implique l'identification basée sur la probabilité de :
sites de recombinaison chromosomique, variations du nombre de copies (sous)chromosomiques, délétions, translocations déséquilibrées ou équilibrées, inversions, amplifications, présence d'allèles à risque pour des troubles héréditaires, erreurs dans la méiose I ou méiose II, anomalies chromosomiques de structure équilibrée ; profils épigénomiques des cellules, mosaicismes, correspondance de l'antigène leucocytaire humain (HLA) ou typage de bruit.

11. Procédé selon la revendication 1, dans lequel l'appel d'ADN discret final implique la détermination du nombre de copies et de l'origine ancestrale des segments maîtres.

12. Procédé selon l'une quelconque des revendications précédentes, dans lequel le regroupement utilise un génome de référence simulé in silico.

13. Procédé selon l'une quelconque des revendications précédentes, dans lequel le regroupement en segments maîtres utilise des informations généalogiques.

14. Procédé selon l'une quelconque des revendications précédentes, dans lequel le regroupement en segments maîtres est basé sur une probabilité ancestrale et dérivé d'informations généalogiques.

15. Procédé selon l'une quelconque des revendications précédentes,
dans lequel le génome d'ADN cible est un génome d'ADN fœtal et
dans lequel ledit génome d'ADN fœtal est dérivé d'un échantillon de fluide obtenu d'une femme enceinte d'un fœtus ayant ledit génome d'ADN fœtal.

16. Procédé selon la revendication 15, comprenant en outre une étape de sélection de taille avant d'effectuer le procédé de séquençage,
dans lequel ladite étape de sélection de taille enrichit des fragments ayant une taille inférieure à 250 paires de bases.
